# EUROPEAN PATENT APPLICATION

(11) **EP 1 953 241 A1**
(43) Date of publication of application: **06.08.2008**
(21) Application number: 06833408.5
(22) Date of filing: 27.11.2006
(51) Int. Cl.: C12Q 1/68

(54) **METHOD OF ANALYZING CHANGE IN PRIMARY STRUCTURE OF NUCLEIC ACID**

(30) Priority: 29.11.2005 JP 2005343763
(71) Applicant: Olympus Corporation, Tokyo 151-0072 (JP)
(72) Inventor: NAGAOKA, Tomonori, Tokyo 166-0001 (JP)
(74) Representative: Wibbelmann, Jobst
(86) International application number: PCT/JP2006/323604
(87) International publication number: WO 2007/063807

(57) **Abstract**

The object of the present invention is to offer a method for analyzing primary structure change of nucleic acid which has quantifiability, which has a high degree of sensitivity and reproducibility, and which can be conducted rapidly and at low cost. The method of analysis of primary structural change of nucleic acid of the present invention includes a process for obtaining a reference nucleic acid having a standard sequence and a target sequence, each sequence being bound by a first ligand that differs depending on type of the sequence to which it binds, and being bound by a second ligand; a process for specifically binding first ligands to receptors supported on a carrier, thereby immobilizing standard sequences and target sequences on the carrier; a process for specifically binding labeled receptors to the second ligands in said nucleic acids which have been immobilized; a process for detecting labels to detect standard sequences and target sequences; a process for obtaining the ratio of standard sequences in the reference nucleic acid and subject nucleic acid, calculating a coefficient which corrects detection values of target sequences, and conducting correction; and a process for confirming an increase/decrease in the quantity of target sequences in the subject nucleic acid relative to target sequences in the reference nucleic acid.

## Description

### TECHNICAL FIELD

The present invention relates to a method of analysis of primary structural change of nucleic acid which is optimal for determining mutations of nucleic acid.

### BACKGROUND ART

In conjunction with completion of decoding of the nucleotide sequences of the human genome, positional information pertaining to the genes in chromosomes has been clarified based on the decoding information. Based on this positional information, research is being actively conducted with a view to practical application of diagnosis concerning various changes in chromosomal structure - i.e., changes in the primary structure of nucleic acid - which become causes of carcinogenesis. In order to comprehensively analyze changes in the primary structure of nucleic acid, there is the array CGH method wherein: micro-arrays are used which spot from several hundred to several thousand and sometimes even several tens of thousands of cDNA clones and BAC clones which have been mapped in chromosomes; nucleic acid is extracted from cells, and subjected to fluorescent labeling with amplification or without amplification of the entirety of the nucleic acid; and heterozygous loss (Loss of Heterozygosity, hereinafter abbreviated as LOH) is analyzed by hybridization.

With respect to analysis by the array CGH method, an analytic method of diagnosing breast cancer has been disclosed (see Patent Literature 1) which uses arrays that arrange clones of genes in 20q13. Changes at specific chromosomal sites - i.e., changes in the primary structure of nucleic acid - can be observed by this method, and applied to cancer diagnosis and the like. A method has also been disclosed (see Non-Patent Literature 1) which uses SNP markers to analyze chromosomal structure with micro-arrays, and to conduct diagnosis of bladder cancer.

On the other hand, as methods of analyzing more restricted chromosomal regions, analytic methods by electrophoresis have been developed which use DNA markers - that is, SNP markers or microsatellite markers - that indicate from several to several tens of polymorphisms. As analytic methods of this type, there has been disclosure of a cancer diagnosis method by instability analysis of microsatellite markers (see Patent Literature 2) and a cancer diagnosis method by loss analysis of SSCP markers (see Patent Literature 3). These methods conduct analysis by measuring differences in the marker length or structure of markers obtained by the PCR method by electrophoresis - when the markers are heterozygous, two fragments appear in the electrophoretic profile. For example, with respect to cancer, a certain marker in a normal tissue of a patient has heterozygosity, while among heterozygous markers in tumor tissue, LOH occurs where one part is lost due to structural anomalies of chromosomes.

With use of such methods, as a result of the loss of the polymorphic marker itself, it is often reported that an anomaly in chromosomal structure has occurred at that position. With respect to the conduct of such analysis, analysis can be conducted with greater data accuracy by registering the various types of DNA markers in public databases, by publicly disclosing primer sequences and the like used when analyzing the markers, and by using these.
Patent Literature 1: U.S. Patent No. 6,268,184 Description
Patent Literature 2: U.S. Patent No. 6,291,163 Description
Patent Literature 3: Japanese Unexamined Patent Application, First Publication No. H9-201199
Non-Patent Literature 1: Cancer Res., 63,2216-2222 (2003)

### DISCLOSURE OF THE INVENTION

### PROBLEMS TO BE SOLVED BY THE INVENTION

However, with respect to the analysis by array CGH method recorded in Patent Literature 1 and Non-Patent Literature 1, time and expense are required to prepare the arrays, and experimental operations are complex, resulting in the problem that it is not practically applied as a method for clinical uses. Moreover, due to the labeling of all nucleic acid extracted from cells and the conduct of hybridization, there is the problem that noise occurs due to cross-hybridization and the like, making it difficult to obtain quantitatively accurate data.
The analytic method which uses polymorphic markers and which employs electrophoresis is also problematic, for not only are there cases of homozygosity due to the genotype of the patient where qualitative analysis is impossible, but also experimental operations are complex and time-consuming, and furthermore it is difficult to accurately quantify targeted genes, with the result that it is impractical as a method for clinical uses.

Accordingly, if it were possible to quantitatively detected DNA markers without relation to patient genotype, detection sensitivity could be improved, and if nucleic acid sequences were mapped on the chromosome even without polymorphisms, it would be possible to quantitatively analyze primary structural changes of nucleic acid sequences. Furthermore, if irregularities could be corrected in the nucleic acid amplification process by PCR or the like, it would be possible to rapidly analyze primary structural changes of nucleic acid with a high degree of accuracy and by simpler operations, and this would be useful as a method for application at clinical uses.

The present invention was made in light of the foregoing circumstances, and its object is to offer a method of analysis of primary structural changes of nucleic acid which is quantitative, which has a high degree of sensitivity and reproducibility, and which can be conducted rapidly and at low cost.

### MEANS FOR SOLVING THE PROBLEMS

In order to solve the aforementioned problems, the present invention has been configured as follows.
(I) A method for analyzing primary structure change of nucleic acid comprising the steps of:
   (1) obtaining a reference nucleic acid having a standard sequence and a target sequence whose existence/non-existence or increase/decrease in amount thereof is to be detected, each sequence being bound by a first ligand that differs depending on type of the sequence to which it binds, and being bound by a second ligand that may be identical or different for each type of the sequence;
   (2) adding first ligands and second ligands to a subject nucleic acid which has a standard sequence and a target sequence whose existence/non-existence or quantity thereof is unknown, in order to obtain nucleic acids which have a structure in which a standard sequence is bound by a first ligand that differs depending on type of the sequence to which it binds, and a second ligand that may be identical or different for each type of the sequence; and if a target sequence is present in the subject nucleic acid, the target sequence is bound by a first ligand that differs depending on type of the sequence to which it binds, and a second ligand that may be identical or different for each type of the sequence;
   (3) preparing a carrier which supports receptors that specifically bind to said first ligands, binding said first ligands to the pertinent receptors, and immobilizing said standard sequences and target sequences on the carrier;
   (4) binding receptors, which are modified with a labeling substance and which specifically bind to second ligands, to the pertinent second ligands in said immobilized standard sequences and immobilized target sequences;
   (5) detecting said labeling substance to detect standard sequences and target sequences;
   (6) obtaining the ratio of standard sequences in a reference nucleic acid and a subject nucleic acid, calculating a coefficient which corrects detection values of target sequences in the subject nucleic acid, and correcting detection values of target sequences in the subject nucleic acid using the pertinent correction coefficient; and
   (7) comparing corrected detection values of target sequences in the subject nucleic acid with detection values of target sequences in the reference nucleic acid to confirm an increase/decrease in the quantity of target sequences in a subject nucleic acid relative to target sequences in a reference nucleic acid.

(II) A method for analyzing primary structure change of nucleic acid comprising the steps of:
   (1) obtaining a reference nucleic acid having a standard sequence and a target sequence whose existence/non-existence or increase/decrease in amount thereof is to be detected, each sequence being bound by a first ligand that differs depending on type of the sequence to which it binds, and being bound by a second ligand that may be identical or different for each type of the sequence;
   (2) adding first ligands and second ligands to a subject nucleic acid which has a standard sequence and a target sequence whose existence/non-existence or quantity thereof is unknown, in order to obtain nucleic acids which have a structure in which a standard sequence is bound by a first ligand that differs depending on type of the sequence to which it binds, and a second ligand that may be identical or different for each type of the sequence; and if a target sequence is present in the subject nucleic acid, the target sequence is bound by a first ligand that differs depending on type of the sequence, and a second ligand that may be identical or different for each type of the sequence;
   (3) preparing a carrier which supports receptors that specifically bind to said second ligands, binding said second ligands to the pertinent receptors, and immobilizing said standard sequences and target sequences on the carrier;
   (4) binding receptors, which are modified with a labeling substance and which specifically bind to first ligands, to the pertinent first ligands in said immobilized standard sequences and immobilized target sequences;
   (5) detecting said labeling substance to detect standard sequences and target sequences;
   (6) obtaining the ratio of standard sequences in a reference nucleic acid and a subject nucleic acid, calculating a coefficient which corrects detection values of target sequences in the subject nucleic acid, and correcting detection values of target sequences in the subject nucleic acid using the pertinent correction coefficient; and
   (7) comparing corrected detection values of target sequences in the subject nucleic acid with detection values of target sequences in the reference nucleic acid to confirm an increase/decrease in the quantity of target sequences in a subject nucleic acid relative to target sequences in a reference nucleic acid.

(III) A method for analyzing primary structure change of nucleic acid comprising the steps of:
   (1) obtaining a reference nucleic acid having a standard sequence and a target sequence whose existence/non-existence or increase/decrease in amount thereof is to be detected, each sequence being bound by a first ligand that differs depending on type of the sequence to which it binds, and being bound by a second ligand that may be identical or different for each type of the sequence;
   (2) adding first ligands and second ligands to a subject nucleic acid which has a standard sequence and a target sequence whose existence/non-existence or quantity thereof is unknown, in order to obtain nucleic acids which have a structure in which a standard sequence is bound by a first ligand that differs depending on type of the sequence to which it binds, and a second ligand that may be identical or different for each type of the sequence; and if a target sequence is present in the subject nucleic acid, the target sequence is bound by a first ligand that differs depending on type of the sequence, and a second ligand that may be identical or different for each type of the sequence;
   (3) preparing a carrier which supports receptors that specifically bind to said first ligands or second ligands, binding said first ligands or second ligands to the pertinent receptors, and immobilizing said standard sequences and target sequences on the carrier, and causing agglutination of the pertinent immobilized standard sequences and immobilized target sequences by an agglutination reaction of the carrier;
   (4) detecting said agglutination product to detect standard sequences and target sequences;
   (5) obtaining the ratio of standard sequences in a reference nucleic acid and a subject nucleic acid, calculating a coefficient which corrects detection values of target sequences in the subject nucleic acid, and correcting detection values of target sequences in the subject nucleic acid using the pertinent correction coefficient;
   (6) comparing corrected detection values of target sequences in the subject nucleic acid with detection values of target sequences in the reference nucleic acid to confirm an increase/decrease in the quantity of target sequences in a subject nucleic acid relative to target sequences in a reference nucleic acid.

(IV) A method for analyzing primary structure change of nucleic acid according to any one of the aforementioned (I) to (III), wherein said second ligands are all uniform.

A method for analyzing primary structure change of nucleic acid according to any one of the aforementioned (I) to (IV), wherein loss of heterozygosity is detected.

(VI) A method for analyzing primary structure change of nucleic acid according to any one of the aforementioned (I) to (V), wherein there is a plurality of said target sequences.

(VII) A method for analyzing primary structure change of nucleic acid according to any one of the aforementioned (I) to (VI), wherein said standard sequences and/or target sequences have nucleotide sequences whose positions on a chromosome are identified as markers.

(VIII) A method for analyzing primary structure change of nucleic acid according to the aforementioned (VII), wherein said nucleotide sequences whose positions on a chromosome are identified as markers are one or more types selected from the group consisting of microsatellite markers, SNP markers, STS markers, EST markers and cDNA markers.

(IX) A method for analyzing primary structure change of nucleic acid according to any one of the aforementioned (I) to (VIII), wherein one of said first and second ligands contain a primer which can amplify said standard sequences and target sequences by a polymerase reaction, while the remaining one of said first or second ligands contain a probe which can hybridize said standard sequences and target sequences, and wherein said standard sequences and target sequences are amplified by a polymerase reaction using the primer, and the amplified standard sequences and target sequences are detected after hybridizing the probe.

(X) A method for analyzing primary structure change of nucleic acid according to the aforementioned (IX), wherein said polymerase reaction is an isothermal amplification reaction.

(XI) A method for analyzing primary structure change of nucleic acid according to any one of the aforementioned (I) to (VIII), wherein said first and second ligands contain a primer capable of amplifying said standard sequences and target sequences by a polymerase chain reaction, and said standard sequences and target sequences are detected after being amplified by a polymerase chain reaction using the pertinent primer.

(XII) A method for analyzing primary structure change of nucleic acid according to any one of the aforementioned (I) to (VIII), wherein said first and second ligands contain probes having nucleotide sequences which are complementary with a portion of said standard sequences and target sequences, and the pertinent probes undergo hybridization and ligation in said standard sequences and target sequences, after which said standard sequences and target sequences which are the ligation products are detected.

(XIII) A method for analyzing primary structure change of nucleic acid according to the aforementioned (XII), wherein said probes are hybridized after amplifying said standard sequences and target sequences by polymerase reaction.

(XIV) A method for analyzing primary structure change of nucleic acid according to any one of the aforementioned (I) to (XIII), wherein said first and second ligands are are polypeptides consisting of two or more amino acid residues.

(XV) A method for analyzing primary structure change of nucleic acid according to any one of the aforementioned (I) to (XIV), which has a process wherein said carrier is a particulate carrier endowed with magnetism.

(XVI) A method for analyzing primary structure change of nucleic acid according to any one of the aforementioned (I) to (XV), wherein the combination of said first ligands and/or second ligands with receptors is a combination of antigen and antibody.

(XVII) A method for analyzing primary structure change of nucleic acid according to any the aforementioned (XVI), wherein said antibody is a monoclonal antibody.

(XVIII) A method for analyzing primary structure change of nucleic acid according to the aforementioned (XVI), wherein said antibody is an antibody obtained by the phage display method.

(XIX) A method for analyzing primary structure change of nucleic acid according to any one of the aforementioned (I) to (XVIII), wherein the detection values of standard sequences and target sequences of said reference nucleic acid are registered in a database, and correction of detection values of target sequences in the subject nucleic acid and comparison of corrected detection values of target sequences in the subject nucleic acid and detection values of target sequences in the reference nucleic acid are conducted using said database.

(XX) A method for determining nucleic acid mutations characterized in that it is determined that amplification of a target sequence in a chromosome has occurred in the case where a corrected detection value of a target sequence in the subject nucleic acid obtained by the method for analyzing primary structural change of nucleic acid according any of the aforementioned (I) to (XIX) is larger than a threshold value.

(XXI) A method for determining nucleic acid mutations characterized in that it is determined that loss of a target sequence in a chromosome has occurred in the case where a corrected detection value of a target sequence in the subject nucleic acid obtained by the method for analyzing primary structural change of nucleic acid according any of the aforementioned (I) to (XIX) is smaller than a threshold value.

### EFFECTS OF THE INVENTION

According to the present invention, as it is possible to quantify a target nucleic acid without relation to polymorphism and mutation, and as it is possible at the time of detection to utilize a reaction of ligand and receptor such as an immune reaction - instead of the conventional hybridization of nucleic acids - in the reaction at the interface of solid and liquid on the carrier, specificity is high, sensitivity is high, and quantifiability is excellent. Furthermore, as operations are simple, and high-temperature conditions are unnecessary, it is possible to conduct analysis at lower cost.

### BRIEF DESCRIPTION OF THE DRAWINGS

Fig. 1 is a schematic view showing one example of the process of the present invention for obtaining a sequence to which ligands are bound.
Fig. 2 is a schematic view showing one example of the process of the present invention for simultaneously obtaining multiple sequences to which ligands are bound.
Fig. 3 is a schematic view showing one example of the process of the present invention for simultaneously obtaining multiple sequences to which ligands are bound and which contain polymorphic sites.
Fig. 4 is a schematic view showing one example of the process of the present invention for obtaining a sequence to which ligands are bound and which contains a SNP marker.
Fig. 5 is a schematic view showing one example of the process of the present invention for simultaneously obtaining multiple sequences to which ligands are bound and which contain polymorphic sites.
Fig. 6 is a schematic view showing one example of the process of the present invention for simultaneously obtaining multiple sequences to which ligands are bound and which contain polymorphic sites.
Fig. 7 is a schematic view showing one example of the process of the present invention for detecting sequences to which ligands are bound.
Fig. 8 is a schematic view showing one example of the process of the present invention for simultaneously obtaining standard sequences and target sequences to which ligands are bound.
Fig. 9 is a schematic view showing one example of the method of the present invention for correcting detection values of a target sequence.
Fig. 10 is a figure which shows the results of correction of detection values of multiple target sequences of the present invention.
Fig. 11 is a figure which shows the results of analysis of primary structural change of nucleic acid in working example 2 of the present invention.

### BEST MODE FOR CARRYING OUT THE INVENTION

Below, the present invention is described in detail.
1. Process for obtaining standard sequences and target sequences modified by ligands
   In the present invention, a subject nucleic acid signifies a nucleic acid which is the subject of analysis for primary structural change. A reference nucleic acid signifies a nucleic acid which is of the same type as the subject nucleic acid, and which has no mutations in nucleotide sequences contained in the below-mentioned standard sequences; it is nucleic acid which constitutes a standard when conducting relative comparison of the degree of primary structural change of the subject nucleic acid.
   There are no particular limitations on the reference nucleic acid or subject nucleic acid so long as the nucleic acid sequences contained therein are of a length which enables conduct of amplification reaction by the PCR method or the like and ligation reaction by ligation. As examples, one may cite all types of DNA and RNA such as genomic DNA, cDNA, synthetic DNA, all-RNA, mRNA, hnRNA, and synthetic RNA. Among these, nucleic acids existing in biological samples are preferably used.

A target sequence signifies a sequence which exists at least once in the aforementioned reference nucleic acid, and which contains a nucleotide sequence subject to analysis whose existence/non-existence or quantity in the subject nucleic acid is unclear.
On the other hand, a standard sequence signifies a sequence which exists at least once in the reference nucleic acid and subject nucleic acid, which has no mutations in its nucleotide sequence, and which serves the purpose of conducting relative comparison of quantities in the reference nucleic acid and subject nucleic acid used in analysis.
In the present invention, a standard sequence and target sequence in the reference nucleic acid and subject nucleic acid are detected, the ratio of the standard sequence in the reference nucleic acid and subject nucleic acid is obtained, a coefficient which corrects a detection value of the target sequence in the subject nucleic acid is calculated, and the detection value of the target sequence in the subject nucleic acid is corrected using the pertinent correction coefficient. An increase/decrease in the quantity of the target sequence in the subject nucleic acid relative to the target sequence in the reference nucleic acid is then confirmed by comparing the corrected detection value of the target sequence in the subject nucleic acid with the detection value of the target sequence in the reference nucleic acid. By this means, it is possible to know whether or not the sequence subject to analysis is contained in the target sequence in the subject nucleic acid, and in the case where it is contained therein, it is possible to compare the degree of mutation such as amplification or loss with an identical nucleotide sequence in the reference nucleic acid.
With respect to the correction coefficient, as a specific example, it may be calculated by dividing the detection value of the target sequence in the reference nucleic acid by the detection value of the target sequence in the subject nucleic acid, and a corrected detection value for the target sequence in the subject nucleic acid is obtained by multiplying the pertinent correction coefficient by the detection value of the target sequence in the subject nucleic acid.

As the standard sequence and target sequence, it is preferable to employ a sequence containing a nucleotide sequence whose position on the chromosome is identified as a marker, and to use these markers as objects of analysis. As such markers, one may preferentially cite one or more from a group consisting of genes, microsatellite markers, SNP markers, STS markers, EST markers, cDNA markers and unique sequences which do not exist in a plurality on the chromosome and which are distinguishable from other sequences.

With the present invention, it is possible to simultaneously analyze multiple target sequences.
In the present invention, it would be difficult to respectively bind a first ligand and second ligand to the standard sequence and target sequence themselves in the reference nucleic acid and subject nucleic acid, immobilize the standard sequence and target sequence on a carrier using these ligands, and provide them for analysis. Consequently, nucleic acid is separately prepared with a structure where the aforementioned ligands are bound to sequences identical to the standard sequence and target sequence, and provided for analysis.

There are no particular limitations on the first ligand and second ligand used in the present invention so long as they are capable of binding to receptors on the below-mentioned carrier. For example, one may cite hapten, biotin, digoxigenin, digoxin, fluorosein, Alexa, Rhodamine (TAMRA), DNP, Texas Red, tetramethylrhodamine, BODIPY FL, dansyl, Lucifer yellow, sugar chain, peptide, protein, polyhistidine, antigen/antibody substances, HA, GST, Tap, Flag, etc. Among these, biotin, digoxigenin, digoxin, fluorosein, and Alexa are optimal, because they are easily obtained as inexpensive commercial products.

As sugar chains and peptides have a high degree of freedom of design of chemical structure, and are easily arranged with more than one type of hapten, various substances can be appropriately selected according to circumstances. As haptens, hydrophilic organic compounds are preferable; as sugar chains, those consisting of two or more monosaccharides are preferable. As peptides, those with an amino acid residue number of 6 or more are preferable, and those with an amino acid residue number of 2 or more are particularly preferable.
In order to conduct detection of nucleic acid using immune reactions, it is acceptable for the combination of a receptor with a first ligand and/or second ligand to be a combination of an antibody with an antigen, and it is also possible to form the specific binding between a ligand and a receptor by an antigen-antibody reaction. Moreover, in the case where an antibody is used as a ligand, the antibody may be a polyclonal antibody, but monoclonal antibodies and antibodies obtained by the phage display method are preferable.

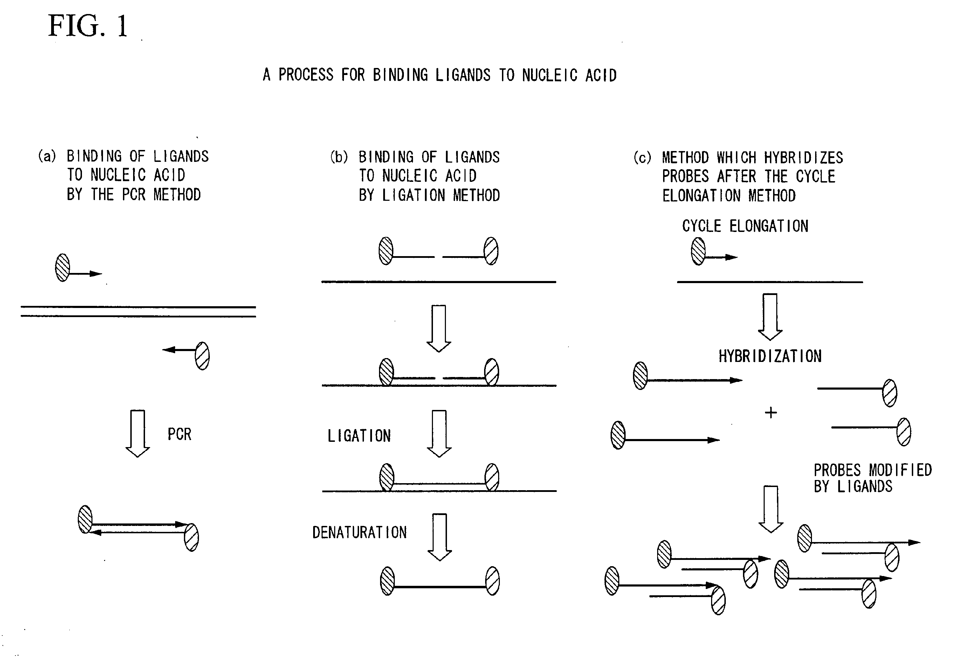

There are no particular limitations on the method for obtaining a standard sequence or target sequence to which a first ligand and second ligand are bound which constitutes the basis of this method, but as preferable methods, one may cite as examples the methods shown in Fig. 1, i.e., the method which amplifies standard sequences and target sequences by polymerase chain reaction (hereinafter "PCR") using a primer modified by the aforementioned ligands (Fig. 1(a)); the method which conducts ligation reaction on a standard sequence or target sequence using a nucleic acid probe modified by ligands (Fig. 1(b)); and the method which hybridizes a nucleic acid probe modified by ligands after the cycle-elongation method using a polymerase reaction (Fig. 1(c)).
When the PCR method is used, not only is it possible to modify standard sequences and target sequences with ligands, but also to amplify their number. A more quantitative amplification of standard sequences and target sequences is possible with use of the cycle elongation method, but as a reverse-side primer is not used, one of the ligands (for example, the second ligand when a primer modified by a first ligand is used) does not bind to the standard sequences and target sequences. Thus, after cycle elongation, a probe modified with one of the ligands is hybridized in the amplified standard sequences and target sequences. It is preferable that the aforementioned polymerase reaction be a constant-temperature amplification reaction. When a ligation reaction is conducted, it is preferable to subject the probe to hybridization after amplification of standard sequences and target sequences by the PCR method, cycle-elongation method, LAMP method or ICAN method. That is, by amplifying in advance the regions where a ligation reaction is conducted in the reference nucleic acid or subject nucleic acid, it is possible to prevent beforehand the binding of the nucleic acid probe to certain similar nucleotide sequences located in other regions of the reference nucleic acid or subject nucleic acid, and by conducting ligation of amplified nucleic acid and denaturing the reaction product, it is possible to obtain standard sequences or target sequences modified by ligands with greater accuracy.

With respect to the first ligand, it is necessary to use different types according to the type of nucleic acid to which binding occurs. With respect to the second ligand, it is necessary to use a type which differs from the first ligand, but it is not necessarily required to use different types according to the type of nucleic acid to which binding occurs. However, by making all second ligands uniform, it is possible to easily conduct B/F separation of standard sequences or target sequences which have been immobilized on a carrier, for example, by means of second ligands, thereby enabling the analysis of the present invention to be conducted more easily and at lower cost.
It is not necessarily required to compose the first and second ligands from one molecule, and it is also acceptable to configure them from two or more molecules.

In the present invention, in order to detect multiple types of standard sequences and/or target sequences via specific binding of ligands and receptors, it is necessary to modify the multiple types of standard sequences and/or target sequences by respectively different ligand combinations. With respect to such associations of nucleic acid and ligands, a variety of methods come to mind. Here, a description will be given of a method for detecting multiple types of standard sequences and/or target sequences by multiplex reaction via specific binding of ligands and receptors.

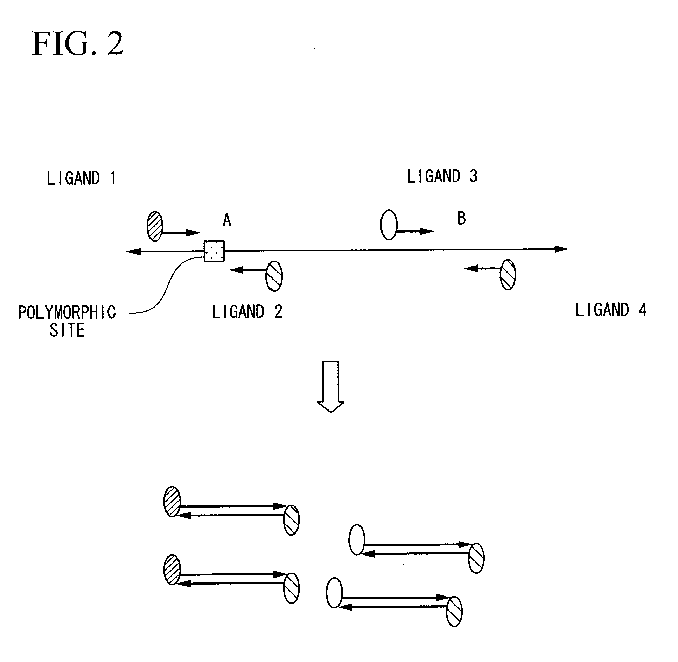

First, using Fig. 2, a description is given of one example of a method for obtaining - by the same reaction system - two types of nucleic acid to which different ligands are bound by the Multiplex PCR (Multiplex Polymerase Chain Reaction) method.

As shown in Fig. 2, four types of primer modified by ligands 1 to 4 are prepared (respectively referred to below as the first to fourth primers). The first primer and second primer are able to amplify sequence A. On the other hand, the third primer and fourth primer are able to amplify sequence B. Here, a single base polymorphism or microsatellite polymorphism or the like may be included at the amplification site. Sequence A and sequence B may be either a standard sequence or a target sequence. There are no limitations on the binding position or quantity of ligands relative to each primer, but binding at the 5' terminal is preferable. It is acceptable that ligand 2 and ligand 4 be uniform. Moreover, it is acceptable that multiple ligands of different types be bound to one primer. It is not necessarily required that a ligand be composed of a single molecule, and a configuration with two or more molecules is also acceptable.
When each primer is hybridized relative to these two amplification sites, the primer binds to the nucleic acid in pairs at a fixed efficiency at each amplification site, enabling conduct of an elongation reaction by polymerase. By conducting hybridization under such simultaneous reaction conditions, it is possible to more quantitatively conduct the association of sequence and ligand.

Subsequently, the sequences are amplified by the PCR method, and sequence A and sequence B modified by ligands are obtained. Each is given its own associations, with sequence A constituting a sequence modified by ligands 1 and 2, and sequence B constituting a sequence modified by ligands 3 and 4. As these associations are conducted under the same reaction conditions, the accuracy of their reaction efficiency ratios is extremely high.
In this instance, so long as sequence A is the standard sequence and sequence B is the target sequence, ligand 1 and ligand 3 correspond to first ligands, and ligand 2 and ligand 4 correspond to second ligands.

The PCR method was cited as a nucleic acid amplification method in this instance, but multiplex amplification may be conducted by a constant-temperature amplification method such as the LAMP method. By using the primer required for a constant-temperature amplification method that binds ligands, a sequence A and sequence B modified by these ligands can be obtained as in the case of the PCR method.

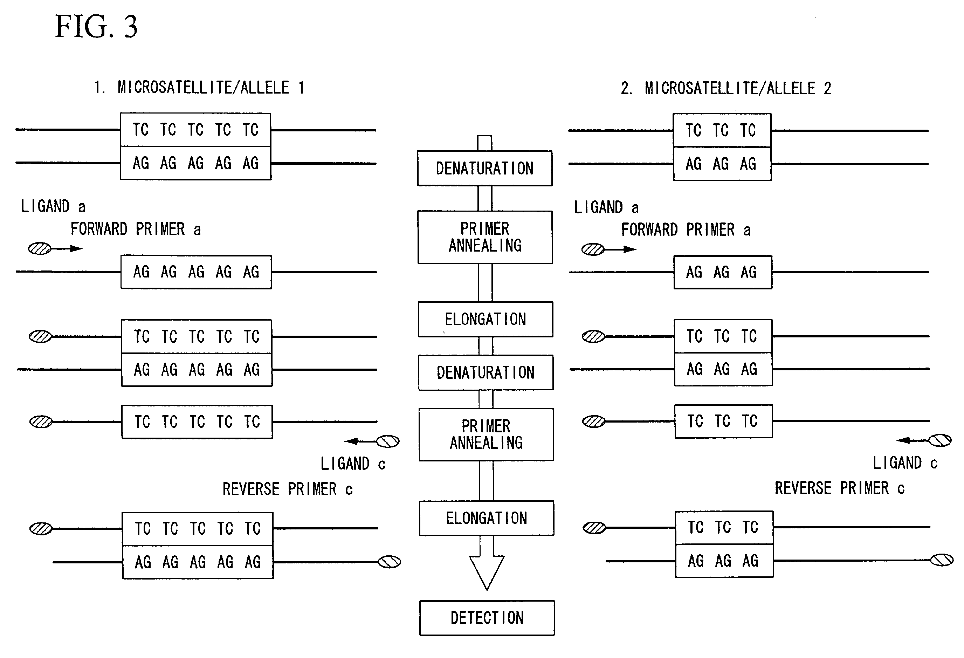

Furthermore, a detailed description is given of the sequences amplified in the foregoing manner with reference to Fig. 3. In the present invention, even if the amplified sequences contain markers indicating polymorphism, the positional information can be obtained without acquiring the polymorphism information.
A primer modified by ligands is prepared, and an amplification reaction is conducted on the subject sample by the microsatellite polymorphism analysis method. In this instance, multiple types of microsatellite repetition numbers exist at the microsatellite polymorphic site of the sample. In a manner including the allyl type of the microsatellite polymorphic site of the sample, forward primer *a* undergoes matching with the upstream of the microsatellite polymorphic site, and amplification is conducted by PCR together with reverse primer *c*. The amplification product is double-strand DNA wherein ligand *a* and ligand *c* are respectively bound to both 5' terminals. Similarly, even in regions in which nucleic acid sequences that do not undergo change have been placed for control purposes, forward primer *b* undergoes matching with upstream of the sequence, and amplification of the sample is conducted by PCR together with reverse primer *c'* (not illustrated in Fig. 3). The PCR product is a double-strand DNA wherein ligand *b* and ligand *c'* are respectively bound to both 5' terminals.
Here, ligand *a* and *b* correspond to first ligands, and ligand *c* and *c'* correspond to second ligands.

In this manner, it is possible to obtain multiple sequences containing microsatellite polymorphic sites modified by ligands from multiple sequences containing microsatellite polymorphic sites.

In analysis of the primary structure of nucleic acid containing a single nucleotide polymorphism (hereinafter "SNP"), one may optimally use, for example, the PCR-SSP method (Polymerase Chain Reaction - Sequence Specific Primer method). The PCR-SSP method is a method which designs sequences so that one of the 3' terminals of a two-type primer overlap with the SNP site of the sequence to be detected, and which controls amplification by PCR according to whether or not there is matching of the 3' terminal of the primer and the SNP site.

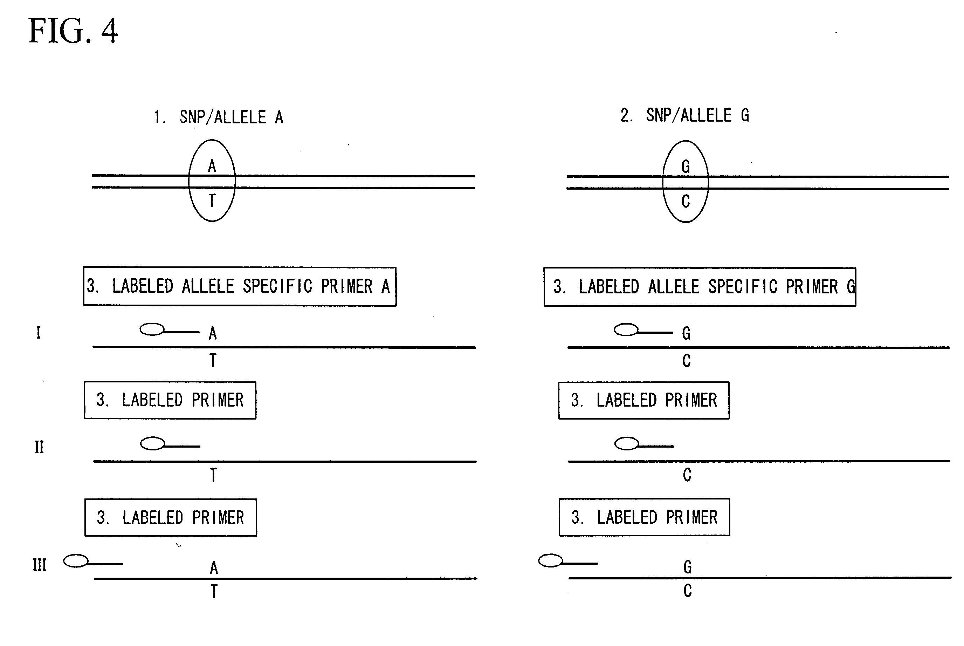

The present invention is capable of observing loss or amplification of sequences where such markers exist without relation to the allele type of SNP sites. Specifically, a method is used which improves on the PCR-SSP method, which is an SNP detection method, and which obtains sequences containing SNP markers modified by a plurality of differing ligands. As one example, a description is given with reference to Fig. 4 for the case where sample SNP sites exist as allyl in the two types of base A (adenine) and base G (guanine). A primer setting method other than the PCR-SSP method is additionally shown.
In Fig. 4, I shows the circumstances pertaining to a primer used in the PCR-SSP method where the 3' terminal of a single type of forward primer modified by ligands terminates by overlapping with the SNP site of the sequence to be detected.
In Fig. 4, II shows the circumstances where a 3' terminal of a single type of forward primer modified by ligands terminates upstream of a single base from the SNP site of the sequence to be detected.
In Fig. 4, III shows a forward primer modified by ligands in an ordinary PCR primer set not for PCR-SSP use that serves to amplify regions where SNPs are constricted. Under these circumstances, there are no limitations on the sites modified by ligands or the number of ligands, but it is preferable to use a 5' terminal as the site modified by ligands. The reverse primer (not illustrated in the drawing) is modified by ligands which differ in type from those of the forward primer. So long as the modifying ligands are different, multiple types may be used. It is not necessarily required that the ligands be composed of one molecule, and configuration from two or more molecules is also acceptable.

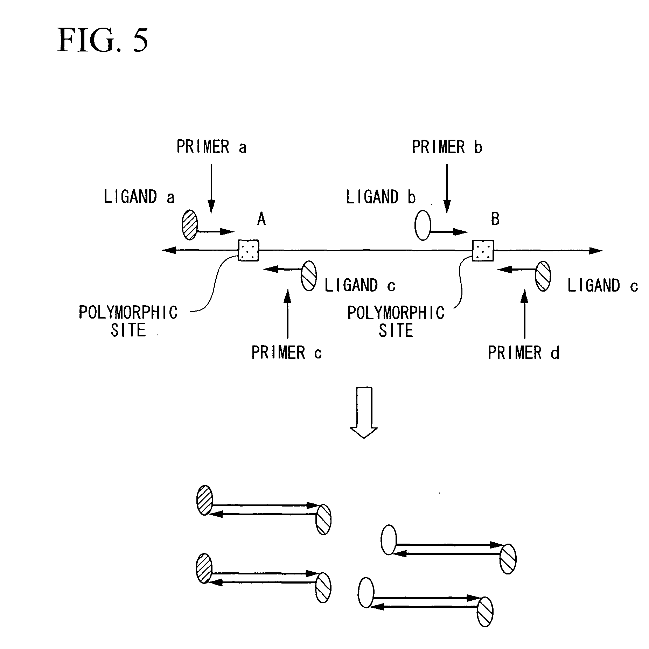

Fig. 5 shows an example of multiplex reaction based on the single reaction of Fig. 3. Four types of primer modified by ligands *a, b,* and c are prepared (primer *a* is modified by ligand *a,* primer *b* is modified by ligand *b,* and primer *c* and primer *d* are modified by ligand *c*). Sample sequence *a* is a target sequence, forward primer *a* undergoes matching on the upstream side of the polymorphic site, and sequence A is amplified by PCR together with reverse primer c. The amplification product is double-strand DNA where both 5' terminals are respectively modified by ligand *a* and ligand *c.* On the other hand, sequence B containing the polymorphic site of a region where the nucleotide sequence does not change is a standard sequence, wherein forward primer *b* similarly undergoes matching on the upstream side of the polymorphic site without relation to the allyl type of the polymorphic site of the sample, and amplification of sequence B occurs by PCR together with reverse primer *d.* The amplification product is double-strand DNA wherein both 5' terminals are respectively modified by ligand *b* and ligand *c.*
According to the foregoing method, it is possible to obtain multiple sequences which are modified by ligands and which contain polymorphic sites.
In this instance, ligand *a* and ligand *b* correspond to first ligands, while ligand *c* corresponds to a second ligand.

According to the above-described method, it is possible to obtain the sequence to be detected as a sequence modified by ligands that differ by type of sequence.

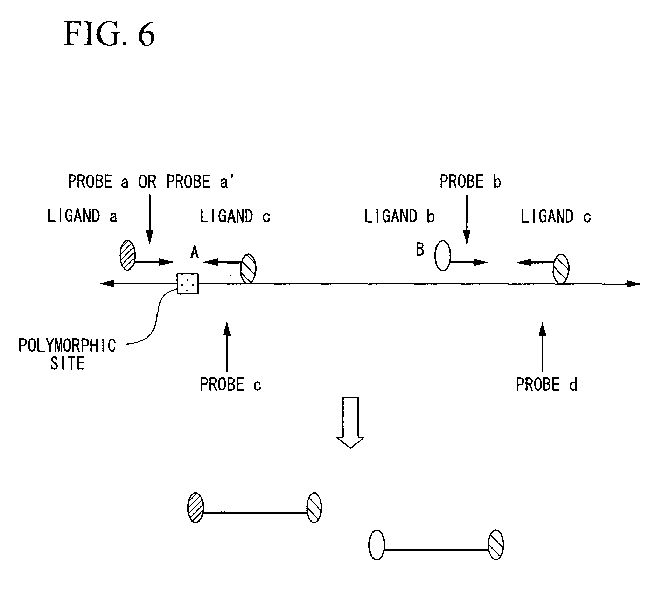

Next, description is given of one example of a method for simultaneously obtaining , by multiplex ligation reaction, two types of sequences to which different types of ligands are bound.
As with the aforementioned PCR method, association of sequence and ligand is also possible by ligation reaction. As shown in Fig. 6, four types of probes modified by ligands *a, b,* and *c* are prepared (probe *a* is modified by ligand *a,* probe *b* is modified by ligand *b,* and probe *c* and probe *d* are modified by ligand *c*). Probe *a* and probe *c* correspond to sequence A, while probe *b* and probe *d* correspond to sequence B. Sequence A and sequence B may be either standard sequences or target sequences. When hybridization is conducted by this multiplex ligation reaction at each sequence site under conditions including the respective probes in the same manner as the multiplex PCR method, it is possible to conduct a ligation reaction by ligase at fixed efficiency where probe pairs bind to the sequence. By conducting hybridization under such simultaneous reaction conditions, it is possible to more quantitatively conduct association of sequence and ligand. Moreover, when the double-strand nucleic acid is denatured to a single strand by a method conducted after the reaction such as rendering alkaline the pH of the buffer liquid used when performing hybridization, the reaction product becomes a product in which single-strand DNA is mixed wherein the two ends of the 5' terminal and 3' terminal are respectively modified by ligand *a* and ligand *c* or ligand *b* and ligand *c.*
As with the PCR method, the method which uses multiplex ligation reaction may be applied even when the sequence subject to detection contains polymorphic sites.
In this instance, ligand *a* and ligand *b* correspond to first ligands, while ligand *c* corresponds to a second ligand.

With ligation reaction, there is no process for amplifying the sample, with the result that the analytic process can be further simplified, and that cost and analysis time can be reduced. However, it is also the case that sensitivity is poor, the degree of freedom in selecting probe design is not high in regions where the homology of sequences is high, and the ligand-modified sequences which are sought cannot be obtained with satisfactory accuracy. Accordingly, in order to enhance sensitivity and improve accuracy, it is preferable to conduct a ligation reaction after the region containing the sequence to be detected has been amplified using a polymerase reaction by a constant-temperature amplification method such as the PCR method, cycle-elongation method, LAMP method or ICAN method.

When doing so, there are no particular limitations on the placement of the probes modified by ligands or the number of ligands, but it is preferable to modify a position which is not in the vicinity of the polymorphic site so as not to impede the reaction of ligase, e.g., to modify the 5' terminal with a probe where the polymorphic site is set at the 3' terminal, and to modify the 3' terminal with the other probe. So long as the modified ligands are different, multiple types may be used. Moreover, it is not necessarily required that the ligands be composed of one molecule, and configuration from two or more molecules is also acceptable.

There are no particular limitations on the ligase used in the present invention, and conventional ligase may be used. For example, one may cite Taq DNA ligase and T4 DNA ligase.

According to the above-described method, it is possible to obtain the sequence that one wishes to detect as a sequence modified by ligands which differ by type of sequence.

There are no particular limitations on the base number of the primers and probes used in the present invention, but it is preferable that they be composed of 3-60 base.

To this point, methods have been described for detecting two types of sequences, but it is also possible to detect three or more types of sequences by the present invention. With respect to three or more types of sequences, in order to conduct a multiplex reaction for each type of sequence, the types of primers or probes increase in number according to the types of the sequences to be detected. For example, if there are three types of sequences to be detected, at least six types of primers or probes are required, and at least four types of ligands are required.
Detection of the sequences may be conducted in separate containers, or simultaneously in the same container.

2. Process for detecting standard sequences and target sequences modified with ligands
Detection of the aforementioned standard sequences and target sequences modified by ligands is conducted by immobilizing the standard sequences and target sequences on a carrier which supports receptors that specifically bind to ligands via the pertinent receptors. For example, in the case where the standard sequences and target sequences are modified by different first ligands and a shared second ligand, carriers which support receptors that specifically bind to the first ligands are prepared for each type of first ligand, the first ligands are made to bind with the receptors, the standard sequences and target sequences are immobilized on the carriers, and these are detected. There are no particular limitations on the sequence detection method, and conventional methods may be applied, but methods which conduct detection by fluorescent dye are preferable.

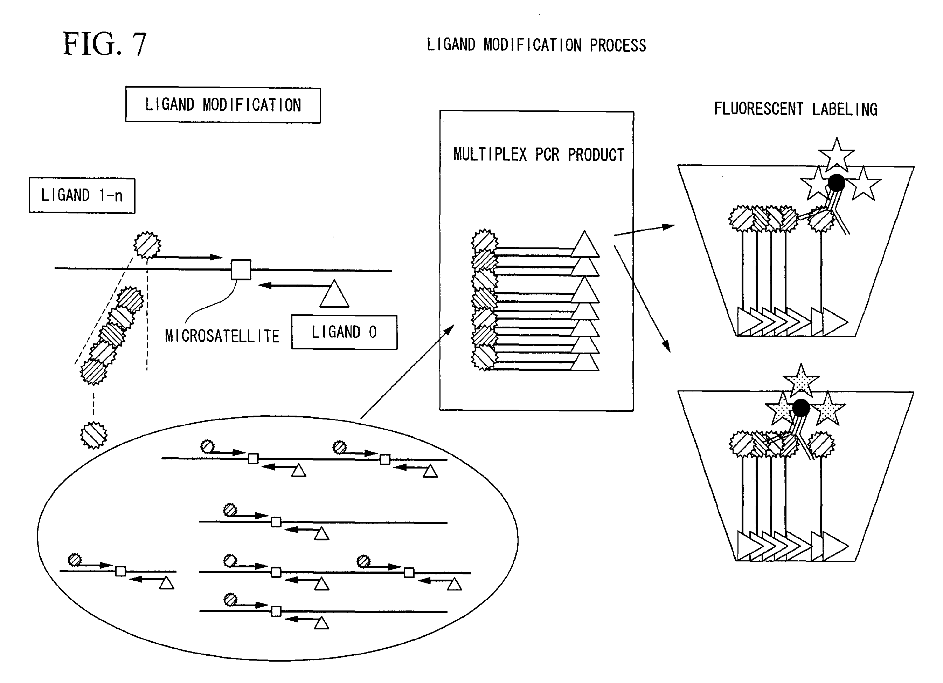

For example, as shown in Fig. 7, carriers which already support receptors that specifically bind to the second ligands are set up in a first container and a second container. Furthermore, the receptor which specifically binds to the first ligand of the standard sequence and the receptor which specifically binds to the first ligand of the target sequence are modified in advance with fluorescent dye. The standard sequence and target sequence modified by ligands bind to the carriers in the first container and the second container via the second ligands. Next, a receptor modified by fluorescent dye binds to the first ligand of the standard sequence in the first container. In addition, a receptor modified by fluorescent dye binds to the first ligand of the target sequence in the second container. Subsequently, it is possible to detect the standard sequence and target sequence by detecting this fluorescent dye using an optical device. The foregoing operations are conducted for both the reference nucleic acid and subject nucleic acid.
As the labeling compound, one may also use material other than fluorescent dye. Moreover, the second ligand may differ in the standard sequence and target sequence, or may be uniform. Furthermore, it is also possible to detect the standard sequence and target sequence in a single container if the labeling substance is changed.

Although not illustrated in the drawings, the following method may also be applied. That is, a carrier which already supports a receptor that specifically binds to the first ligand of the standard sequence is set up in a first container, and a carrier which already supports a receptor that specifically binds to the first ligand of the target sequence is set up in a second container. Furthermore, the receptors which specifically bind to the second ligands are modified in advance with fluorescent dye. The standard sequence modified by ligands and the target sequence modified by ligands bind to the carriers in the first container and the second container respectively via the first ligands. Next, the receptors modified by fluorescent dye bind to the second ligands in the first container and the second container. Subsequently, it is possible to detect the standard sequence and target sequence by detecting this fluorescent dye using an optical device. The foregoing operations are conducted for both the reference nucleic acid and subj ect nucleic acid.
Here, also, one may also use material other than fluorescent dye as the labeling compound. Moreover, the second ligand may differ in the standard sequence and target sequence, or may be uniform. Furthermore, in the case where carriers are used which support receptors that specifically bind to the first ligands of both the standard sequence and the target sequence, it is also possible to detect the standard sequence and target sequence in a single container if one uses second ligands and labeling substances which differ in the standard sequence and target sequence.

On the other hand, it is also acceptable to have ligands identical to the first ligand of the standard sequence and/or the first ligand of the target sequence which are already supported by carriers. In this case, the receptors undergo specific binding to the aforementioned supported ligands. Next, the first ligands of the standard sequence and/or target sequence modified by ligands bind to the aforementioned receptors.
A carrier which support ligands has a better shelf life than a carrier which supports receptors, which is a major advantage when practically applying a detection method.
The ligands which are already supported by carriers may also be second ligands.

Detection of the standard sequence and target sequence may also be conducted by enzyme reaction. There are no particular limitations on the enzyme used in this case, and conventional enzymes may be used. As examples of such enzymes, one may cite alkaline phosphatase, peroxidase, etc. In this case, for example, the receptors which specifically bind to the aforementioned second ligands are modified in advance with enzyme, and the chemiluminescence which occurs from the reaction of the substrate with this enzyme is detected.
Receptors which are modified in advance with enzyme may also specifically bind with first ligands.

The detection process by chemiluminescence will be described in further detail citing an example. First, carriers are compelled to support receptors. Instead of this, it is also acceptable to use carriers which already support receptors. Next, the standard sequence and/or target sequence modified by ligands bind to the aforementioned carriers via the specific binding between the receptors and second ligands. At this time, the unbound free standard sequence and/or target sequence on the carriers are removed by B/F separation. Subsequently, antibodies modified by the aforementioned enzyme bind to the first ligands of the standard sequence and/or target sequence which are bound to the carrier. At this time, the labeling antibodies which are unbound and free are removed by B/F separation. Finally, chemiluminescence occurs as a result of enzyme-substrate reaction, and the nucleic acid subject to detection is identified by detection thereof. This method may also be applied to the detection methods for the aforementioned sequences where ligands are supported on carriers.

It is also possible to use carriers which are compelled to support receptors that specifically bind to the second ligands of a standard sequence and target sequence, and to simultaneously detect the standard sequence and target sequence. Particularly in the case where the second ligands are uniform, both standard sequence and target sequence can easily bind to carriers which support receptors that specifically bind to second ligands. With respect to both the standard sequence and target sequence which are bound to carriers, the first ligands are in an unbound state.
Accordingly, it is possible to modify in advance receptors which specifically bind to first ligands with labeling substances of different types in the standard sequence and target sequence, and to simultaneously detect the standard sequence and target sequence using these labeling substances.

On the other hand, as another method of detecting standard sequences and target sequences modified by ligands, one may cite the method which uses agglutination reaction.
Specifically, for example, it is possible to have receptors pertaining to first ligands and receptors pertaining to second ligands supported in advance on separate surfaces or the same surface of dispersive microparticles of polystyrene latex beads or the like, and to detect the standard sequence and target sequence by an agglutination reaction of this carrier.

In the present invention, agglutination refers to that which occurs due to the cross-linking reaction of dispersive microparticles with a standard sequence or target sequence. For example, when a reaction is engendered respectively involving a standard sequence or target sequence in which first ligands of different types and second ligands which may be uniform are bound, a first microparticle in which multiple receptors which specifically bind to the aforementioned first ligands are bound, and a second microparticle in which multiple receptors which specifically bind to the aforementioned second ligands are bound, the first microparticle and second microparticle are ligated due to the binding of one standard sequence or target sequence with the first microparticle and second microparticle. Furthermore, numerous microparticles mutually ligate to produce agglutination when multiple standard sequences or target sequences bind to a single microparticle, and these standard sequences or target sequences bind to other microparticles. In the present invention, the agglutination product produced in this manner is measured by the spectrophotometric method.

By apportioning carriers which support receptors according to the respective ligand combinations corresponding to the standard sequences and target sequences subject to detection into multiple containers, and by dispensing samples containing the standard sequences and target sequences subject to detection into each container, it is possible to detect specific sequences in the respective containers by causing agglutination thereof.

The microparticles used in the present invention are dispersive microparticles, and signify, for example, particles which disperse in a solution like colloidal particles. Latex particles are optimal for use, but one is not limited thereto. When measuring turbidity by an immunoturbidimetric assay device, it is preferable that the particle size of the dispersive microparticles be 80-300 nm.

The latex particles used in the present invention are mainly composed of polystyrene, and they are copolymerized with methacrylic acid in order to enhance hydrophilic properties and dispersion properties. With respect to latex particles, there is the plain type which has no functional groups on the surface, and the functional group types which has functional groups. The charge on the surface of the plain type has a negative charge due to the presence of methacrylic acid, and is capable of ionic bonding with regions possessing the positive charge of proteins. It is also capable of hydrophobic bonding. As protein is adsorbed merely by mixing the plain type of latex particles with protein, protein immobilization operations are easy.

Latex particles having functional groups are designed so that carboxyl groups, amino groups and the like are exposed on the surface, and various types of the functional group type of latex particles may be used. As methods for causing receptors to bind to functional group type latex particles, one may cite the EDAC method which causes amino groups to bond with carboxylic acid in aqueous carbodiimide, the method which causes bonding of carboxylic acid and amino groups after first mixing EDC and NHS, the method which cross-links fellow amino acid groups using bipolar linkers, the method which bonds proteins by activated aldehyde groups and tosyl groups, etc. With respect to the present invention, any of the conventional methods beginning with the aforementioned methods may be used. Among these, the EDAC method is preferable, and it is, for example possible to use EDAC manufactured by Sigma Co. Even with microparticles other than latex particles, it is possible to bind receptors to the pertinent microparticles by conventional methods.

As receptors which bind to dispersive microparticles, receptors are used which specifically bind to ligands which are bound to the standard sequences and target sequences subject to detection. As such receptors, one may use, for example, antibodies, lectin, streptavidin, Ni and the like, but one is not limited thereto, and one may use various substances which are mentioned below. Among these, proteins - particularly antibodies - are optimal for use. Apart from the aforementioned methods, there are no particular limitations on the method for binding these receptors to microparticles, and it is acceptable to appropriately apply conventional methods according to the type of microparticles. Moreover, it is also acceptable to use dispersive microparticles in which receptors have been bound in advance.

As a method of detecting agglutination product, one may cite the method which uses the binding of a single type of receptor to one dispersive microparticle. As at least two types of ligands are bound to the standard sequences and target sequences in the present invention, it is possible to prepare at least two types of dispersive microparticles relative to one type of standard sequence or target sequence. That is, one may use a first microparticle to which only a receptor corresponding to a first ligand is bound and a second microparticle to which only a receptor corresponding to a second ligand is bound.

As another method, one may cite the method which uses the binding of two or more types of receptors to one dispersive microparticle. With this method, among the two types of ligands bound to one standard sequence or target sequence, it is preferable to have one dispersive microparticle to which is bound the receptor corresponding to one of the ligands and a receptor other than the receptor corresponding to the other ligand. That is, it is preferable to conduct design so that both of the two types of ligands of the standard sequence or target sequence do not bind to one dispersive microparticle.

It is possible to conduct binding and obtain an agglutination product by causing a reaction of the first and second microparticles to which receptors are bound with the standard sequence and/or target sequence to which ligands are bound. This reaction may be conducted by simultaneously mixing two solutions respectively containing the first and second microparticles with a solution containing the standard sequence and/or target sequence, or it may be sequentially conducted by mixing the two solutions respectively containing the first and second microparticles one after the other. The solution containing both the first and second microparticles may then be mixed with the solution containing the standard sequence and/or target sequence.

When a first ligand bound to the standard sequence or target sequence binds with the first microparticle, and when the second ligand binds with the second microparticle, the first and second microparticles are ligated by the binding of one standard sequence or target sequence to the first and second microparticles. Furthermore, agglutination of microparticles occurs due to the mutual ligation of numerous microparticles, which results from the bonding of multiple standard sequences or target sequences to one microparticle and from the bonding of these standard sequences or target sequences with other microparticles.

By this means, when standard sequences or target sequences exist in a sample, it is possible to product agglutination of microparticles, and to detect the standard sequences or target sequences. Here, the presence or absence of microparticle agglutination can be determined by measuring light absorbance by the spectrophotometric method.
That is, a single microparticle causes almost no scattering of near-infrared light, but agglutinated microparticles have a larger apparent radius than a single microparticle, and cause scattering of near-infrared light. Accordingly, it is possible to measure the degree of agglutination of microparticles by using a photometer to measure the luminance of transmitted light relative to incoming light of the near-infrared region. When doing so, as microparticles with a particle size of 300 nm or less optimally scatter near-infrared light when agglutinated, it is preferably to use microparticles with a particle size of 300 nm or less in the present invention, and use of microparticles with a particle size of 80-300 nm is still more preferable.

With respect to the solution containing the microparticles, it is preferable that the final concentration assuming the case where agglutination does not occur after mixing be within a range of 0.01-1 in terms of OD values. Furthermore, it is preferable that the post-agglutination OD value be 3 or less. In order to avoid the occurrence of bias in the binding of ligands and receptors when a solution containing microparticles is mixed with a solution containing the standard sequences and/or target sequences, it is preferable that the volume of one of the solutions not fall 20% below the volume of the other solution, and that agitation be rapidly conducted by a Lutex mixer or by pipetting. There are no particular limitations on the buffer liquid used during mixing, but it is preferable to use a buffer liquid which accelerates agglutination.

The other mixing conditions may be appropriately selected according to the employed ligand-receptor combinations. For example, in the case where antigen-antibody is used as the ligand-receptor combination, it is preferable to conduct mixing and agitation for approximately five minutes at 30°C.

When presenting the reaction solution after mixing for spectrophotometric measurement, as microparticles which have not agglutinated do not scatter near-infrared light, there is no need to conduct B/F separation, and the reaction system can be presented for direct measurement. When preparing the standard sequences and/or target sequences by the PCR method, in cases where there is a risk that measurement errors will occur due to unreacted primer or unreacted nucleotides to which ligands are bound, it is preferable to separate these. As a method of separation, one may cite, for example, the method which binds biotin to the ligands, and which conducts BF separation using magnetized particles to which avidin or streptavidin is bound.

Next, a method is described for quantifying the standard sequences and target sequences used in the method of the present invention. When conducting quantification, the aforementioned standard sequences and target sequences to which ligands are bound are used, and solutions containing these sequences in various concentrations are prepared. The sequence solution of each concentration is made to react with microparticles according to the aforementioned method to produce an agglutination product, and light absorbance is determined by the spectrophotometric method. Based on the pertinent measurement results, a calibration curve is prepared from the relation of light absorbance and the concentration of standard sequence and target sequence. Based on this calibration curve, these sequence concentrations are calculated from the results of absorbance determination pertaining to the standard sequences and target sequences subject to detection. By this means, it is possible to conduct quantification of standard sequences and target sequences.

In the case where samples are used which contain multiple types of standard sequences and/or target sequences, first ligands which differ according to the type of these sequences and second ligands which may be uniform are compelled to bind to these sequences, and one supplies for reaction only dispersive microparticles to which receptors are bound that specifically bind with the ligands bound to the standard sequences and/or target sequences to be detected, thereby enabling simultaneous detection of the desired sequences from a sample containing multiple types of standard sequences and/or target sequences.

With respect to the ligands used relative to one standard sequence or target sequence, it is acceptable to use not only two types, but also three or more types, and it is acceptable to use dispersive microparticles to which receptors are bound that specifically bind to the respective types of ligands.

Next, a description is given of the receptors and carriers used in the present invention.
The receptors of the present invention refer to substances to which first and second ligands specifically bind. For example, in the case where biotin is selected as a ligand, avidin is the receptor, and in the case where hapten is selected as a ligand, an anti-hapten antibody is the receptor. There are no particular limitations on anti-hapten antibodies, but antibodies which have undergone affinity purification, monoclonal antibodies, and antibodies obtained by the phage display method are preferable, because of enhanced specificity and detection accuracy. Moreover, the combination of ligands and receptors may be combinations of antigens and antibodies, and the aforementioned specific binding of ligands and receptors may be formed by antigen-antibody reaction. By detecting standard sequences or target sequences using immune reaction in this manner, high-precision detection becomes possible.

The carriers of the present invention signify support media capable of sustaining the aforementioned receptors. There are no particular limitations on the material or form of the carriers. As the material, one may cite, for example, glass, silicon, metal, metal oxide, resin, rubber, and ceramics. In the case where linkers are used in order to cause binding of receptors, it is preferable that the carrier surface have active groups such as hydroxyl groups, carboxyl groups, amino groups or thiol groups in order to engender reaction with the linking agent. In the case of a metal surface, as coordinate bonding with thiol groups is possible, there may be cases where it would be acceptable to have no active groups. With respect to form, one may cite, for example, board-like and particulate forms. Specifically, a slide glass form (ca. 25 mm x 75 mm x 1 mm) is preferable in the case where a DNA microarray scanner capable of high-sensitivity fluorescent measurement is used, while a microplate shape (ca. 86 mm x 128 mm) is preferable in the case where a chemiluminscence plate reader is used. Furthermore, it does not matter whether the carrier is liquid-permeable or liquid-impermeable. As liquid-permeable carriers are often made of porous material, surface area can be enlarged, and the immobilization amount of receptors such as antibodies can be increased, and as the degree of freedom is enhanced in the direction of movement of the liquid, there is the advantage that the automation of reaction is facilitated. However, as the range of material selection is narrowed, and as improvement of detection equipment is sometimes required, it is necessary to conduct appropriate selection according to circumstances.

There are no particular limitations on the method for supporting receptors on a board-like carrier, and conventional methods may be applied. For example, one may cite the methods for conducting support by physical adsorption, chemical bonding, etc. Here, physical adsorption is bonding by intermolecular attraction such as van der Waal's force or hydrophobic bonding, while chemical bonding refers to covalent bonding formed between functional groups on the carrier and functional groups on the receptors. Chemical bonds may be formed via linkers between the carrier and the receptors. As the employed linkers, one may select an optimal type according to the material and surface condition of the carrier and the type of antibody.
The method which conducts support by physical adsorption has the merit that the process is simple. The method which conducts support by chemical bonding has the merits that receptors can be supported more stably on the carrier, and that when, for example, anti-hapten antibodies are used as receptors, it is advantageous for maintaining their activity. As to which method is to be adopted, selection may be appropriately conducted by taking into consideration the receptors and carriers which are employed.

Here, one example of a method of preparation of a carrier which supports anti-hapten antibodies as receptors is shown. However, the present invention is not limited to the following contents, and any carrier may be used so long as it fulfills the above-described contents.
In the case where low-fluorescent silica glass is used as the carrier, the pertinent glass is thoroughly delipidated with alcohol, after which it is washed with pure water. Next, a solution is prepared so that the concentration of aminopropyltrimethoxysilane (manufactured by Shin-Etsu Silicones) in ethanol is 3 mass percent. The glass is immersed in this solution at room temperature, and a reaction is conducted for 1 hour while stirring. Cleaning is sequentially conducted with ethanol and pure water, after which drying is conducted for 20 minutes at 130°C.

Next, EDC (manufactured by Pierce Co.) is dissolved in MES buffer liquid so that the concentration is five mass percent, the treated glass is immersed in this solution, and a reaction is conducted for 1 hour while stirring. After the reaction, cleaning is sequentially conducted with MES buffer liquid and pure water, and drying is conducted by the spin-dry method at room temperature.

The anti-hapten antibodies use FITC as hapten *a* and DIG as hapten *b.* As the anti-hapten antibodies corresponding thereto, a solution is prepared in which anti-FITC antibodies and DIG antibodies subjected to affinity purification are dissolved in MES buffer liquid so that the concentration is 5 mg/ml, and this solution is spotted at different positions on the glass. For spot application, for example, a spotting device SPBIO (manufactured by Hitachi Software Co.) of the sensing pin type is used. After spotting, a reaction is conducted for one hour at room temperature, after which immersion is conducted for one hour at room temperature in PBS buffer liquid in which BSA (bovine serum albumen) is dissolved to a concentration of 0.1 mass percent. After immersion, cleaning is sequentially conducted with PBS buffer liquid and with PBS buffer liquid of 1/10 concentration, drying is conducted by the spin-dry method, and storage is conducted in a dry condition in cool and dark place.

In this manner, a carrier supporting receptors may be prepared. As not merely two types, but also three or more types of anti-hapten antibodies may be immobilized, it is possible to further increase the types of standard sequences or target sequences subject to detection.

In the present invention, a particulate may be used as the carrier, and the aforementioned receptors may be supported thereon.
There are no particular limitations on the material of a particulate carrier, but resin particulates are often used. For example, one may cite natural rubber, styrene, styrene copolymer, polyurethane, acrylic resin, etc. It is also possible to mix magnetic material with the particulate carrier. By imparting magnetism to a particulate carrier, handling of the particulate carrier is facilitated. As the method for mixing magnetic material with a particulate carrier, conventional methods may be applied.

As the method for supporting receptors on a particulate carrier, one may apply the method for supporting receptors on a board-like carrier.

In the case where biotin is used as the ligands, and avidin ore streptavidin as the receptors, one may use commercially available magnetic beads to immobilize the avidin or streptavidin.

In the present invention, after receptors are supported on the aforementioned carrier, it is possible to immobilize a standard sequence or target sequence on the carrier by causing at least one of the ligands of a standard sequence or target sequence modified by ligands to bond with the aforementioned receptors.

A method for supporting receptors on a carrier is shown below with presentation of specific ligands, primers, probes and sequences subject to detection.
Using hapten as the ligands, an example is shown below of a method of conducting analysis by causing a standard sequence or target sequence modified by hapten to bind to a board-like carrier.
A standard sequence modified by hapten *a* as the first ligand and hapten *c* as the second ligand and a target sequence modified by hapten *b* as the first ligand and hapten c as the second ligand are respectively dissolved in MES buffer liquid, dripped onto a board-like carrier, and made to undergo reaction for two hours at 37°C, after which the carrier was washed with MES buffer liquid. Anti-hapten *a* antibodies and anti-hapten *b* antibodies are supported on the carrier. At this time, the standard sequence modified by hapten *a* in the solution dripped onto the carrier binds to the anti-hapten *a* antibodies on the carrier, and the target sequence modified by hapten *b* binds to the anti-hapten *b* antibodies on the carrier. As a result, hapten c which is the unbound second ligand exists in the standard sequence and target sequence immobilized on the carrier.

Anti-hapten c antibody solution labeled with Cy5 which is a fluorescent dye is dissolved in MES buffer liquid so that the concentration is 10 µg/ml, this solution is dripped onto the carrier on which the standard sequence and target sequence are immobilized, and reaction is conducted for two hours at 37°C. Subsequently, the carrier is sequentially washed after the reaction with MES buffer liquid and with MES buffer liquid of 1/10 concentration, and dried by the spin-dry method. By this means, Cy5 binds to the hapten c in the standard sequence and target sequence immobilized on the carrier via the anti-hapten c antibodies.

Next, the carrier is set in GenePix 4000B (manufactured by Axon Instruments Co.), is scanned at an excitation wavelength of 635 nm and a detection wavelength band of 650 nm to 690 nm, and the fluorescent signals on the carrier are measured. In the scan image, it is possible to detect the SNPs of the subject nucleic acid by conducting a combined analysis of positional information pertaining to the carrier and fluorescent intensity.

Instead of anti-hapten c antibodies labeled with aforementioned Cy5, one may also use anti-hapten c antibodies labeled with enzyme, e.g., with alkaline phosphatase. In this case, the hapten c in the reference nucleic acid and subject nucleic acid and the anti-hapten c antibodies labeled with alkaline phosphatase undergo binding, and the carrier is washed, after which drip infusion of a substrate - e.g., CDP-Star (manufactured by Calbio Chem. Co.) solution - is conducted, the amount of chemiluminescence on the carrier is measured by Luminescencer (manufactured by Atto Co.), and combined analysis with positional information pertaining to the carrier is conducted to detect the SNPs of the subject nucleic acid.

It is also acceptable to support anti-hapten *a* antibodies and anti-hapten *b* antibodies on the carrier via hapten *a* and hapten *b.* For example, by causing the carboxylic groups at the C terminal of hapten to bind to linkers on the carrier in accordance with the aforementioned method, the hapten can be made to bind to the carrier. If a mixed solution of anti-hapten *a* antibodies and anti-hapten *b* antibodies is dripped onto this carrier, if a reaction is conducted for two hours at 37°C, and if cleaning is conducted according to the aforementioned method, the anti-hapten *a* antibodies and anti-hapten *b* antibodies can be supported on the carrier. Thereafter, it is sufficient to immobilize the standard sequence and target sequence on the carrier and to conduct analysis by the same methods described above.

Next, an example is shown below of a method where the same standard sequence and target sequence recorded in the above-described example using a board-like carrier are bound to a particulate carrier endowed with magnetism, and analyzed.

In accordance with the conventional methods, anti-hapten c antibodies are supported on a particulate carrier endowed with magnetism, this carrier is dissolved in phosphate buffer liquid, a standard sequence and target sequence are mixed therewith, and these sequences bind to the particulate carrier. As the particulate carrier is endowed with magnetism, it is possible to move the carrier inside the container and to collect it at one place in the solution by applying a magnetic force from the outside. For example, the solution is removed in a state where the carrier is fixed to a side wall of the container using a magnet, a new phosphate buffer liquid is added, the magnet is then removed, and the carrier is dispersed in this newly added phosphate buffer liquid. By repeating this process several times, it is possible to remove unreacted sequences and by-product in the solution (BF separation process). After the B/F separation process, the solution in which the carrier is uniformly dispersed in a phosphate buffer liquid is divided in two, and pipetted into separate containers. The amounts which are pipetted are determined according to the amount of standard sequence and target sequence subject to measurement and the amount of employed hapten. As the standard sequence and target sequence bind to the carrier via hapten c, hapten *a* is in an unbound state in the case of the standard sequence, and hapten *b* is in an unbound state in the case of the target sequence. Accordingly, it is possible to detect the standard sequence and target sequence by using labeled anti-hapten *a* antibodies and anti-hapten *b* antibodies.

Enzyme-labeled anti-hapten *a* antibodies and enzyme-labeled anti-hapten *b* antibodies are separately introduced into the respective solutions containing the aforementioned carrier which was pipetted in two, and a reaction is engendered. After the reaction, the aforementioned BF separation process is conducted, and the unreacted enzyme-labeled anti-hapten antibodies are removed. When doing so, alkaline phosphatase and peroxidase may be optimally used as the enzyme, but the present invention is not limited thereto.

Finally, the compound constituting the substrate of the enzyme is mixed into the respective containers, and constant-time reactions are conducted, after which luminescence intensity, changes in light absorbance and the like are measured, thereby enabling simultaneous detection of the standard sequence and target sequence subject to detection.

It is also possible to simultaneously detect the standard sequence and target sequence subject to detection by using anti-hapten *a* antibodies and anti-hapten *b* antibodies labeled with fluorescent dye, instead of enzymes, and by measuring fluorescence intensity.

It is also possible to detect the standard sequence and target sequence subject to detection by respectively preparing a carrier compelled to support anti-hapten *a* antibodies and a carrier compelled to support anti-hapten *b* antibodies in separate containers, dividing in two the standard sequence and target sequence modified by hapten, pipetting them into these containers, and mixing.

In the case where a particulate carrier not endowed with magnetism is used instead of a particulate carrier endowed with magnetism, one may cite the methods in the BF separation process where: (i) the carrier is captured by a filter; (ii) the carrier is precipitated by still standing, centrifugal separation or the like; (iii) in the case where a carrier of large particle size is used, as it is not possible to exert suction by pipette, solution exchange is conducted as is.

### 3. Analysis of measurement data

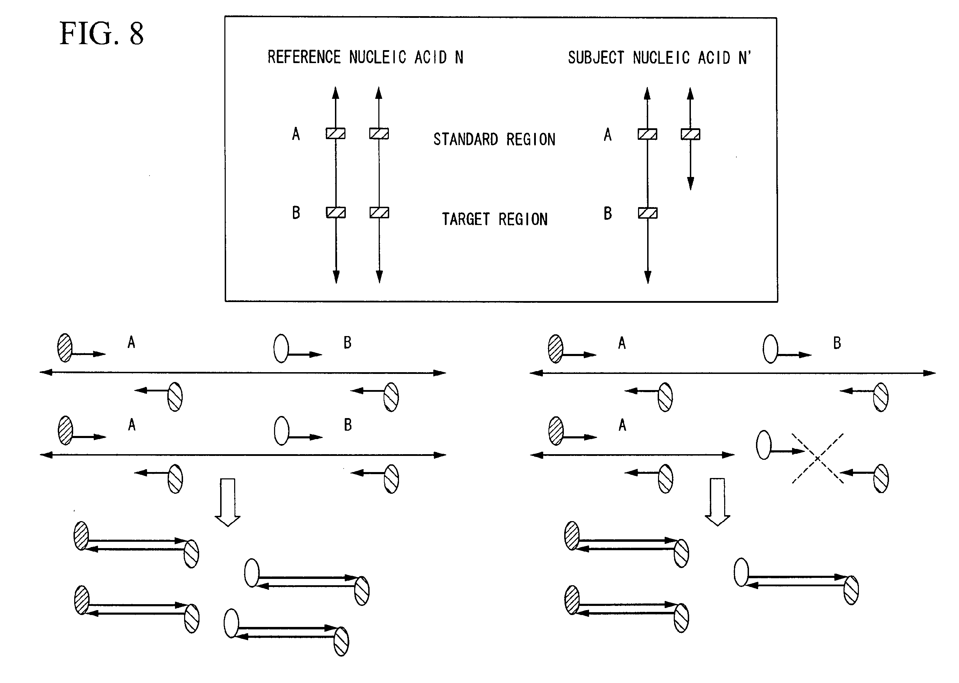

Next, a method is described for analyzing primary structural change (here, LOH) of nucleic acid from the detection data of standard sequences and target sequences in reference nucleic acid and subject nucleic acid. The method of the present invention for analyzing primary structural change of nucleic acid obtains the ratio of a standard sequence in the reference nucleic acid and a standard sequence in the subject nucleic acid to compute a correction coefficient, and corrects detection values of the target sequence in the subject nucleic acid using the pertinent correction coefficient. The method is described using Fig. 8.
In Fig. 8, A shows standard regions, and B shows target regions. With respect to the reference nucleic acid N, the nucleotide sequences are normal sequences in standard regions A and target regions B. With respect to the subject nucleic acid N', the standard regions A are normal, but LOH occurs in the target region B. First, with respect to the reference nucleic acid N, the nucleotide sequences containing the standard regions A are considered as the standard sequences, and the nucleotide sequences containing the target regions B are considered as the target sequences, and the standard sequences and target sequences are amplified by the PCR method according to the methods previously described, under the same reaction system. The difference in the conversion efficiencies of standard regions A and target regions B - i.e., the difference in the conversion efficiencies of the standard sequences and target sequences by the PCR method - are constant, and are dependent on the initial abundance ratio.
Similarly, with respect also to the subject nucleic acid N', the standard sequences and target sequences are amplified by the PCR method under the same reaction system.

When the signals of the obtained amplification products are measured, in the subject nucleic acid N' where LOH has occurred in target region B, the ratio of the amplification product amount of target region B relative to the amplification product amount of standard region A is lower than in the case of the reference nucleic acid N.

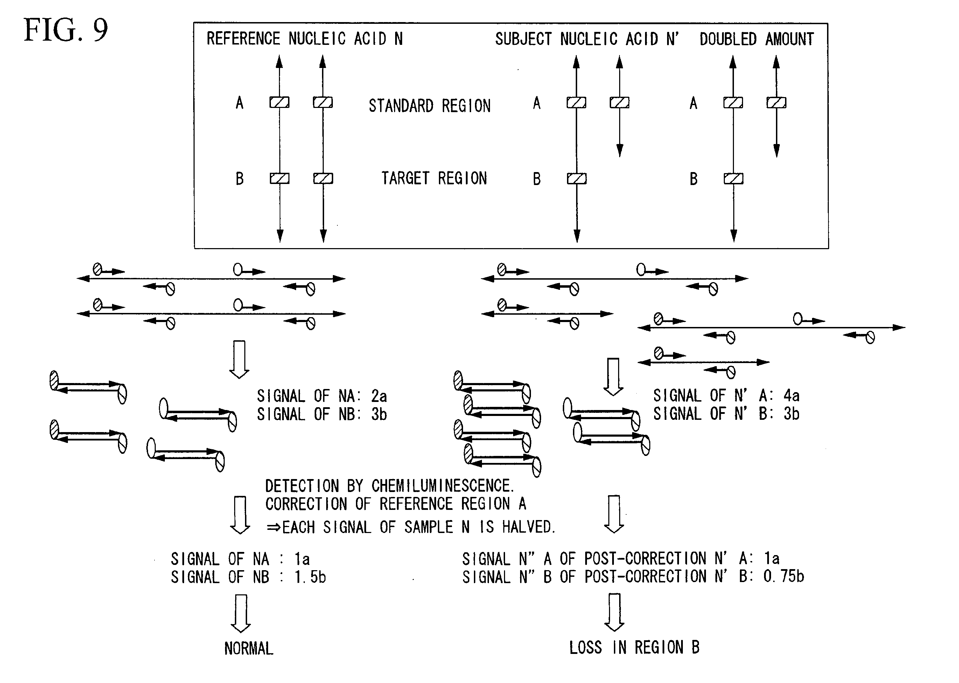

Here, the method of determining the measurement results is described in further detail using Fig. 9. Fig. 9 shows the case where the amount of the subject nucleic acid N' used in measurement is twice the amount of the reference nucleic acid N.
As a result of measurement, the signal intensity of the standard sequences (NA) of the reference nucleic acid N was 2a, and the signal intensity of the target sequences (NB) was 3b, while the signal intensity of the standard sequences (N'A) of the subject nucleic acid N' was 4a, and the signal intensity of the nucleic acid (N'B) was 3b.
The correction coefficient may be calculated by dividing NA by N'A, and is in this instance 0.5. When this coefficient is multiplied by N'B, the correction value of N'B is 1.5b. When the signal intensities of target sequences is compared between the reference nucleic acid N and the subject nucleic acid N', NB : corrected N'B = 2 : 1. That is, assuming a LOH threshold value of 1.5b, the amplification product of the target sequence of the subject nucleic acid N' is reduced by half compared to the ordinary type of reference nucleic acid N, and it is clear that LOH occurs in target region B.
The calculation method of the correction coefficient and the correction method are not limited to the foregoing. As shown in Fig. 9, with the reference nucleic acid N and subject nucleic acid N', it is also acceptable to correct NA and N'A to the identical value, and to compare the NB and N'B obtained at that time.

A description was given here of the case where LOH occurs in a target region B of a subject nucleic acid N', but in the case where amplification occurs in the target region B, one can confirm the fact that NB < corrected N'B by the same analysis.

In addition, a method was shown here for amplifying and obtaining a standard sequence and target sequence by the PCR method, but, as stated above, it is also acceptable to apply a method for obtaining a standard sequence and target sequence by the cycle elongation method, LAMP method, or ligation method.

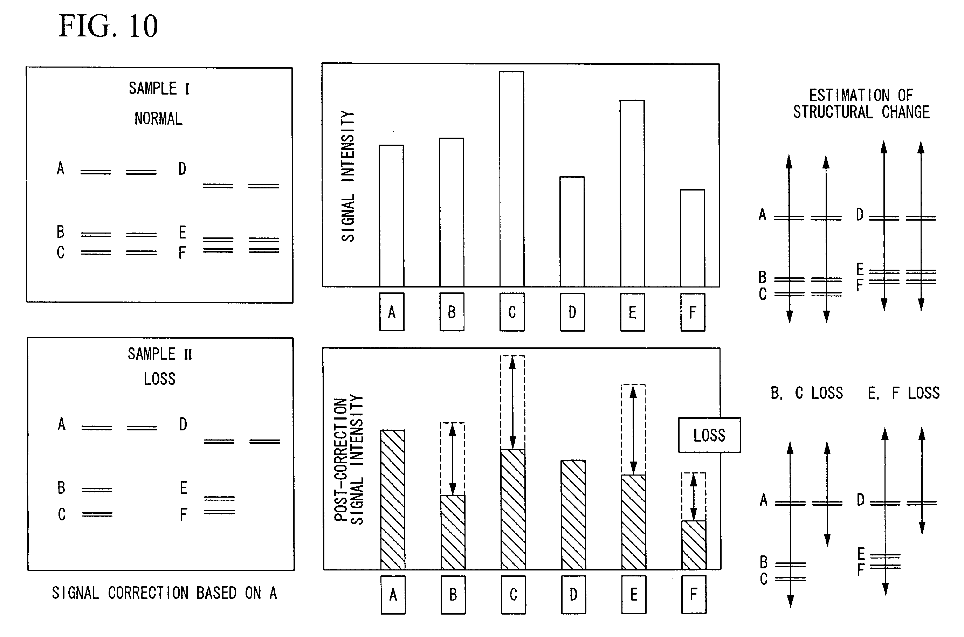

Furthermore, an example was stated here of analyzing a single standard sequence and a single target sequence, but in the case of a plurality of standard regions and target regions, one may also analyze a plurality of corresponding standard sequences and target sequences by increasing the types of ligands and receptors. For example, as shown in Fig. 10, it is also possible to simultaneously analyze a single standard region A and five target regions B-F.

Moreover, the method described here was for measuring regions containing SNP markers or microsatellite markers, but the present invention is not limited thereto. It may also be applied to the detection of regions containing other markers, such as STS markers, EST markers, and cDNA markers, and enables analysis of primary structural changes of nucleic acid such as chromosomes from changes in the quantity thereof. That is, by establishing primers that can amplify regions containing such markers, or by establishing probes in the nucleotide sequences of regions containing such markers, the aforementioned methods can be applied as is with respect to other processes. Moreover, even if one does not use primer sequences already recorded in a database, it is also acceptable to newly design and use primers.

As previously stated, it is not necessarily required to conduct continuous detection of the standard sequences and target sequences in a reference nucleic acid and subject nucleic acid. For example, in the case where one conducts detection of standard sequences and target sequences in a subject nucleic acid in multiple repetitions under the same conditions, with the method of analysis of primary structural change of nucleic acid of the present invention, it is sufficient to conduct detection of standard sequences and target sequences in the reference nucleic acid once, record the pertinent detection values in a database, and use the pertinent database in analyzing the subject nucleic acid from the second time onward to conduct correction of the detection values of target sequences as well as comparison of the detection values of target sequences in the reference nucleic acid with the pertinent corrected detection values.

Using the results of analysis of primary structural change of nucleic acid obtained by the analytic method of the present invention described above, it is possible to determine abnormalities of target sequences in chromosomes. That is, if the quantity of target sequences in the subject nucleic acid is larger than the quantity of target sequences in the reference nucleic acid, it can be determined that amplification of the target sequences has occurred in the chromosome, and if less than the quantity of target sequences in the reference nucleic acid, it can be determined that loss of target sequences has occurred in the chromosome. Specifically, for example, it is sufficient to conduct the determination according to whether the corrected detection value of a target sequence in the aforementioned subject nucleic acid is greater than or less than the threshold value. Here, as the threshold value, it is acceptable to use a detection value of a target sequence in the reference nucleic acid.

### (Examples)

The present invention is described in further detail below by specific working examples. However, the present invention is not limited in any way by the following working embodiments.
The nucleic acid sequences of the respective sequence numbers constituting the model sample used in these examples may be either single-strand or double-strand.

### Example 1

### 1. Reagent preparation

The following reagents were prepared.
Sample 1/reference nucleic acid solution 1: a sample containing sequences represented by Seq. ID No. 5 and Seq. ID No. 6 at 2 nM each, which is a sample possessing a normal type of chromosomal structure.
Sample 2/subject nucleic acid solution 2: a sample containing 2 nM of a sequence represented by Seq. ID No. 5 and 1 nM of a sequence represented by Seq. ID No. 6, which is a sample possessing an abnormal type of chromosomal structure in which LOH occurs in the region of Seq. ID No. 6.
PCR reagent: "Accuprime Super Mix II" manufactured by Invitrogen Co., catalog No. 12341-012.
PCR primer: combination of the primers shown below.
- Primer wherein the 5' terminal of a primer *a* expressed by Seq. ID No. 1 is modified by FITC
- Primer wherein the 5' terminal of a primer *b* expressed by Seq. ID No. 2 is modified by biotin
- Primer wherein the 5' terminal of a primer *c* expressed by Seq. ID No. 3 is modified by digoxigenin (Dig)
- Primer wherein the 5' terminal of a primer *d* expressed by Seq. ID No. 4 is modified by biotin
The standard sequence was amplified by primers *a* and *b,* and the target sequence was amplified by primers *c* and *d*.
Magnetized particulate carrier 1: "Dynabeads M-280 streptavidin" (manufactured by Dynal Co., catalog No. 112.06) to which streptavidin was bound
Magnetized particulate carrier 2: "Dynabeads M-280 streptavidin" (anufactured by Dynal Co., catalog No. 112.06) to which anti-FITC antibodies was bound
Enzyme labeling antibodies: antibodies to which "Anti-Digoxigenin (rabbit polyclonal antibody with ALP)" manufactured by Dako Co., catalog No. D5105 was bound.
Luminescent substrate: "CDP-Star substrate" manufactured by Wako Co., catalog No.
69086.

### 2. Reaction

Reactions were conducted by the following procedure using the aforementioned reagents according to three types of sample combination.
- Sample 1 or 2 :: 1 µl
- PCR reagent :: 10 µl
- Primer prepared at 1 µM :: 1 µl each (total of 4 µl)
- Pure water :: 5 µl
- Total :: 20 µl
PCR solutions were prepared with the above content, and the following reactions were conducted by thermal cycler. (1) 94°C, 30 minutes; (2) 55°C, 30 seconds; (3) 72°C, 30 minutes; (4) 35 cycles of the aforementioned (1) to (3).
After the reactions, with respect to sample 1, in order to conduct measurement of the standard sequence amplified by primers *a* and *b* and the target sequence amplified by primers *c* and *d*, the reaction liquid was pipetted into two microtubes in the amount of 5 µl each. To one tube was added 5 µl of the magnetized particulate carrier 1, engendering a reaction for five minutes at room temperature. To the other tube was added 5 µl of the magnetized particulate carrier 2, engendering a reaction for five minutes at room temperature. After reaction, B/F separation was conducted twice with a phosphate buffer solution with respect to each tube, 20 µl of enzyme labeling antibody diluted 100 times were respectively added, and reaction was conducted for 60 minutes at room temperature. Next, after respectively conducting BF separation three times with a phosphate buffer solution, 100 µl of luminescent substrate were respectively added, and reaction was conducted for one minute at room temperature. After reaction, the carrier was transferred to a 96-well black plate for measurement, and integrative measurement was conducted for ten seconds in a luminescence amount measuring device (Luminescencer-JNRII, manufactured by Atto Co.).
The same operations were conducted with respect to sample 2, and measurement of the two sequences amplified by primers *a* and *b* and primers *c* and *d* was conducted.

### 3. Measurement results and findings

Among the two amplification products of sample 1, the measured value pertaining to the luminescent amount of the standard sequence amplified by primers *a* and *b* was 400000, while the measured value pertaining to the target sequence amplified by primers *c* and *d* was 320000. On the other hand, among the two amplification products of sample 2, the measured value pertaining to the standard sequence amplified by primers *a* and *b* was 840000, while the measured value pertaining to the target sequence amplified by primers *c* and *d* was 330000. When the measured value pertaining to the standard sequence of sample 1 was set to 1, the measured value pertaining to the standard sequence of sample 2 was 2.1. With respect to amplification of the target sequence with primers *c* and *d,* efficiency was kept fixed with that of amplification of the standard sequence with primers *a* and *b.* Based on this value, when correction was conducted by dividing the measured value of the target sequence of sample 2 amplified by primers c and *d* by 2.1, it was found that the measured correction value was 15714, which was half the measured value of the target sequence of sample 1, reflecting the abundance of the target sequence. That is, this working example shows that LOH analysis by the present invention is possible.
In the present working example, measurement of the signals of sample 1 and sample 2 was continuously conducted, and with respect to data pertaining to the standard sequence and target sequence in the reference nucleic acid of sample 1, the same data is obtained each time so long as measurement is conducted under the same conditions. Consequently, as stated above, by obtaining this data once and by registering it in a database, it is possible to repetitively use the data in the database every time in the case where, for example, measurement of sample 2 is conducted on multiple occasions under the same conditions, and it is not necessarily required to continuously conduct measurement of the signals of sample 1 and sample 2.

### Example 2

Nucleic acid containing a single nucleotide polymorphism was detected by ligation reaction. Before conducting ligation reaction, in order to exclude similar sequences and enhance detection sensitivity, samples possessing nucleotide sequences of 80 bp and 85 bp were made on the assumption that the nucleotide sequences around the aforementioned single nucleotide polymorphic site is later subjected to amplification by the multiplex PCR method. Particles endowed with magnetism were used in the carrier, and sequences were detected by enzyme substrate reaction. When template DNA exists in sufficient quantity and when it is possible to conduct direct detection of ligation, conduct of direct detection without performing amplification by the PCR method enables more accurate reflection of the abundance ratio of nucleic acid.

### 1. Reagent preparation

The following reagents were prepared.
Sample 1: a sample containing sequences represented by Seq. ID No. 7 and Seq. ID No. 8 at 100 nM each, which is a sample possessing a normal type of chromosomal structure.
Sample 2: a sample containing 200 nM of a sequence represented by Seq. ID No. 7 and 100 nM of a sequence represented by Seq. ID No. 8, which is a sample possessing an abnormal type of chromosomal structure in which LOH occurs in the region of Seq. ID No. 8.
   With respect to the sequence of Seq. ID No. 8, the base at the position of r is A or G. Furthermore, the region of number 25 to number 61 from the 5' terminal of the sequence represented by Seq. ID No. 7 is a probe hybridization site, and the region of number 19 to number 54 from the 5' terminal of the sequence represented by Seq. ID No. 8 is a probe hybridization site.
   Probe: a combination of the probes shown below.
   - Probes in which the 5' terminal of a probe *a* expressed by Seq. ID No. 9 is modified by digoxigenin (Dig), and the 3' terminal of a probe *b* expressed by Seq. ID No. 10 is modified by biotin, and the 5' terminal is phosphorylated
   - Probes in which the 5' terminals of a probe c expressed by Seq. ID No. 11 and a probe *d* expressed by Seq. ID No. 12 is modified by FITC, and the 3' terminal of a probe e expressed by Seq. ID No. 13 is modified by biotin, and the 5' terminal is phosphorylated
      Ligase: "Taq DNA Ligase" manufactured by BioLabs Co., catalog No. M0208S.
      Magnetized particulate carrier: "Dynabeads M-280 streptavidin" manufactured by Dynal Co., catalog No. 112.06.
      Enzyme labeled antibodies: "Anti-Digoxigenin (rabbit polyclonal antibody with ALP" manufactured by Dako Co., catalog No. D5105.
      Luminescent substrate: "CDP-Star substrate" manufactured by Wako Co., catalog No. 69086.

### 2. Reaction

Reactions were conducted by the following procedure using the aforementioned reagents according to three types of sample combination.

| | |
|---|---|
| Sample 1 or sample 2 | : 1 µl |
| Taq Ligase buffer liquid (supplied by kit) | : 10 µl |
| 40 U/µl of Taq Ligase | : 0.5 µl |
| Respective probes prepared at 10 nM: | 1 µl each (total of 5 µl) |
| Pure water | : 20.5 µl |
| Total | : 30 µl |

The foregoing were respectively prepared, and the following reactions were conducted in an incubator. (1) 95°C, 5 minutes; (2) 58°C, 15 minutes.
After the reactions, magnetized particulate carrier was added in the amount of 5 µl, and reaction was conducted for five minutes at room temperature. After reaction, BF separation was conducted twice with a phosphate buffer, 20 µl of enzyme labeling antibody diluted 100 times were added to each, and reaction was conducted for 60 minutes at room temperature. After respectively conducting BF separation reaction three times with a phosphate buffer, 100 µl of luminescent substrate were respectively added, and reaction was conducted for one minute at room temperature. After reaction, the carrier was transferred to a 96-well black plate for measurement, and integrative measurement was conducted for ten seconds in a luminescence amount measuring device (Luminescencer-JNRII, manufactured by Atto Co.).

### 3. Measurement results

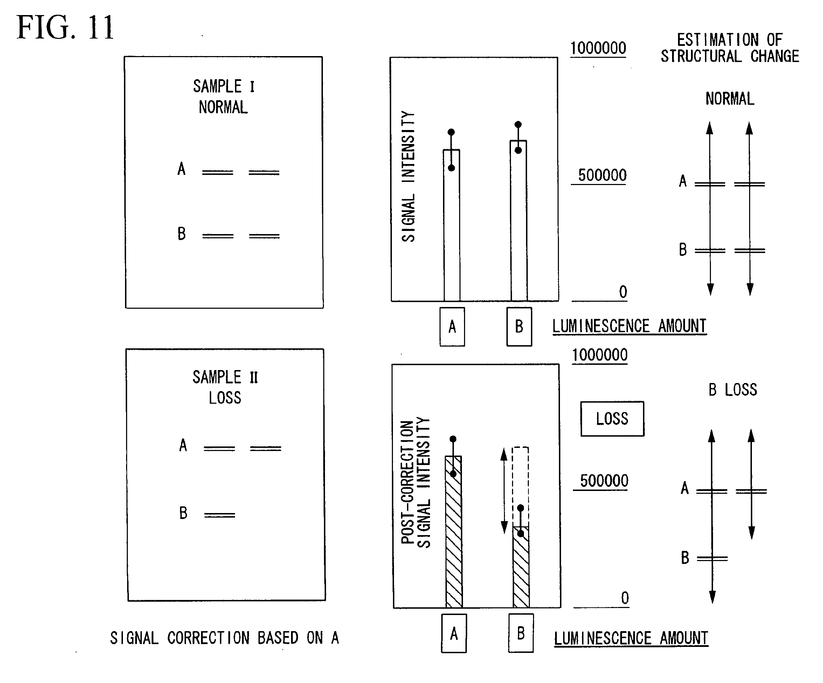

Fig. 11 shows the results of 9 repetitions of the aforementioned reactions conducted with respect to each of the samples. Fig. 11 is a graph which shows average values of the luminescence amounts for the respective combinations of sample and probe, and it is clear that the results according to sample sites are obtained as luminescence amounts. Sample 1 corresponds to the reference-nucleic acid, and sample 2 corresponds to the subject nucleic acid. Furthermore, Seq. ID No. 7 corresponds to the probe standard sequence, and Seq. ID No. 8 corresponds to the target sequence. With the reference nucleic acid, the ratio of the average values of luminescence amounts is standard sequence A : target sequence B = 1 : 1.2, and with the subject nucleic acid, it is standard sequence A : target sequence B = 1 : 0.6, so that the average luminescence amount value of the target sequence is significantly lower than the threshold value at 1.2 ± 10%, thereby clearly indicating detection of sample 2 which is a model possessing an abnormal type of chromosomal structure wherein target sequence B of the subject nucleic acid - i.e., the region of Seq. ID No. 8 - has undergone LOH.
Thus, the method of the present invention using ligation reaction is very useful, because quantification of samples can be conducted with particular clarity and because LOH differentiation which has been difficult with conventional methods is possible.

### Example 3

Detection of the nucleic acid used in Example 1 was conducted by using dispersive microparticles and measuring agglutination of the pertinent microparticles.

### 1. Reagent preparation

The following reagents were prepared.
Sample 1 and sample 2 of Example 1
PCR reagent: "Accuprime Super Mix II" manufactured by Invitrogen Co., catalog No. 12341-012.
PCR primer: combination of the primers shown below.
- Primer wherein the 5' terminal of a primer *a* expressed by Seq. ID No. 1 is modified by hapten *a* expressed by Seq. ID No. 14
- Primer wherein the 5' terminal of a primer *b* expressed by Seq. ID No. 2 is modified by hapten *c* expressed by Seq. ID No. 16
- Primer wherein the 5' terminal of a primer *c* expressed by Seq. ID No. 3 is modified by hapten *b* expressed by Seq. ID No. 15
- Primer wherein the 5' terminal of a primer *d* expressed by Seq. ID No. 4 is modified by hapten *c* expressed by Seq. ID No. 16
The standard sequence was amplified by primers *a* and *b,* and the target sequence was amplified by primers *c* and *d*.
The primers modified by hapten were prepared by the following procedure. That is, 100 µl of a 500 µM hapten solution was added to 100 µl of a 100 µM primer wherein the 5' terminal is modified by carboxylic groups, and underwent condensation reaction by mixing for 30 minutes at 30°C. After termination of the reaction, purification was conducted with a gel filter cartridge, and primer modified by hapten was eluted with 100 µl of a phosphate buffer liquid (pH 7.4).

### 2. Reaction

Reactions were conducted by the following procedure using the aforementioned reagents according to three types of sample combination.

| | |
|---|---|
| Sample 1 or 2 | : 5 µl |
| PCR reagent | : 50 µl |
| Primer prepared at 1 µM | : 5 µl each (total of 20 µl) |
| Pure water | : 25 µl |
| Total | : 100 µl |

PCR solutions were prepared with the above content, and the following reactions were conducted by thermal cycler. (1) 94°C, 30 minutes; (2) 55°C, 30 seconds; (3) 72°C, 30 minutes; (4) 40 cycles of the aforementioned (1) to (3).

### 3. Preparation of antibody-binding latex

Next, the dispersive microparticle carrier shown below was obtained which binds receptors, which specifically bind with the ligands bound to standard sequences and target sequences, to dispersive microparticles.
Dispersive microparticle carrier 1: a carrier to which mouse antibody for hapten *a* ("300 nm carboxyl-type latex particles (CMP) G0201 (0.104 meq COO/g)" manufactured by JSR Co.) was bound
Dispersive microparticle carrier 2: a carrier to which mouse antibody for hapten *b* ("300 nm carboxyl-type latex particles (CMP) G0201 (0.104 meq COO/g)" manufactured by JSR Co) was bound.
Dispersive microparticle carrier 3: a carrier to which mouse antibody for hapten c ("300 nm carboxyl-type latex particles (CMP) G0201 (0.104 meq COO/g)" manufactured by JSR Co.) was bound
The specific preparation method is as follows. That is, 400 µl of 100 mM phosphate buffer liquid (pH 7.4) containing mouse antibody (manufactured by Wako Pure Chemical Industries, Ltd.) at a concentration of 60 µg/ml was added to 100 µl of polystyrene latex (1 mass % of solid content; manufactured by Sekisui Chemical Co.) with an average particle size of 0.4 µm, and was agitated for 1 hour at 4°C. Next, 2 ml of 100 mM phosphate buffer liquid (pH 7.4) containing bovine serum albumin (manufactured by Sigma Co.) at a concentration of 1 mass % was added, and agitation was conducted for 2 hours. This liquid was subjected to centrifugal separation at 16000 rpm for 20 minutes at 10°C. 5 ml of 100 mM phosphate buffer liquid (pH 7.4) containing bovine serum albumin (manufactured by Sigma Co.) at a concentration of 0.3% (w/v) was added to the obtained precipitate, and the latex was suspended to prepare the antibody-binding latex liquid.

### 4. Measurement method

Measurement of the agglutination product was conducted using an automatic biochemical analyzer (AU640, manufactured by Olympus Co.). The measurement conditions were set to a temperature of 37°C and wavelength of 660 nm. With respect to 150 µl of a 0.2% (w/v) antibody-binding latex solution, addition and mixing were conducted in the proportion of 10 µl of sample liquid and 50 µl of streptavidin-binding latex liquid. After addition of the aforementioned latex liquid, the amount of change in light absorbance was measured from 120 seconds to 600 seconds.

### 5. Measurement results and findings

Among the two amplification products of sample 1, the measured value pertaining to the light absorbance of the standard sequence amplified by primers *a* and *b* was 0.9, while the measured value pertaining to the target sequence amplified by primers *c* and *d* was 0.7. On the other hand, among the two amplification products of sample 2, the measured value pertaining to the standard sequence amplified by primers *a* and *b* was 0.9, while the measured value pertaining to the target sequence amplified by primers *c* and *d* was 0.3. When the measured value pertaining to the standard sequence of sample 1 was set to 1, the measured value pertaining to the standard sequence of sample 2 was also 1. With respect to amplification of the target sequence with primers *c* and *d*, efficiency was kept fixed with that of amplification of the standard sequence with primers *a* and *b.* Based on this value, when correction was conducted by dividing the measured value of the target sequence of sample 2 amplified by primers *c* and *d* by 1, it was found that the measured correction value was 0.3, which was less than half the measured value of the target sequence of sample 1, reflecting the abundance of the target sequence. That is, this example shows that LOH analysis by the present invention is possible.

From the foregoing, it was confirmed that when analysis of primary structural change of nucleic acid is conducted, the present invention is a method of analysis which is quantifiable, which has a high degree of sensitivity and reproducibility, and which can be conducted rapidly and at low cost.

### INDUSTRIAL APPLICABILITY

According to the method of the present invention, as it is possible to detect desired target sequences in a subject nucleic acid conveniently, rapidly and accurately, it is useful for the simplification and acceleration of, for example, clinical testing, and can further be widely applied from simple tests to fully automated analysis.

## Claims

1. A method for analyzing primary structure change of nucleic acid comprising the steps of:
(1) obtaining a reference nucleic acid having a standard sequence and a target sequence whose existence/non-existence or increase/decrease in amount thereof is to be detected, each sequence being bound by a first ligand that differs depending on type of the sequence to which it binds, and being bound by a second ligand that may be identical or different for each type of the sequence;
(2) adding first ligands and second ligands to a subject nucleic acid which has a standard sequence and a target sequence whose existence/non-existence or quantity thereof is unknown, in order to obtain nucleic acids which have a structure in which a standard sequence is bound by a first ligand that differs depending on type of the sequence to which it binds, and a second ligand that may be identical or different for each type of the sequence; and if a target sequence is present in the subject nucleic acid, the target sequence is bound by a first ligand that differs depending on type of the sequence to which it binds, and a second ligand that may be identical or different for each type of the sequence;
(3) preparing a carrier which supports receptors that specifically bind to said first ligands, binding said first ligands to the pertinent receptors, and immobilizing said standard sequences and target sequences on the carrier;
(4) binding receptors, which are modified with a labeling substance and which specifically bind to second ligands, to the pertinent second ligands in said immobilized standard sequences and immobilized target sequences;
(5) detecting said labeling substance to detect standard sequences and target sequences;
(6) obtaining the ratio of standard sequences in a reference nucleic acid and a subject nucleic acid, calculating a coefficient which corrects detection values of target sequences in the subject nucleic acid, and correcting detection values of target sequences in the subject nucleic acid using the pertinent correction coefficient; and
(7) comparing corrected detection values of target sequences in the subject nucleic acid with detection values of target sequences in the reference nucleic acid to confirm an increase/decrease in the quantity of target sequences in a subject nucleic acid relative to target sequences in a reference nucleic acid.

2. A method for analyzing primary structure change of nucleic acid comprising the steps of:
(1) obtaining a reference nucleic acid having a standard sequence and a target sequence whose existence/non-existence or increase/decrease in amount thereof is to be detected, each sequence being bound by a first ligand that differs depending on type of the sequence to which it binds, and being bound by a second ligand that may be identical or different for each type of the sequence;
(2) adding first ligands and second ligands to a subject nucleic acid which has a standard sequence and a target sequence whose existence/non-existence or quantity thereof is unknown, in order to obtain nucleic acids which have a structure in which a standard sequence is bound by a first ligand that differs depending on type of the sequence to which it binds, and a second ligand that may be identical or different for each type of the sequence; and if a target sequence is present in the subject nucleic acid, the target sequence is bound by a first ligand that differs depending on type of the sequence, and a second ligand that may be identical or different for each type of the sequence;
(3) preparing a carrier which supports receptors that specifically bind to said second ligands, binding said second ligands to the pertinent receptors, and immobilizing said standard sequences and target sequences on the carrier;
(4) binding receptors, which are modified with a labeling substance and which specifically bind to first ligands, to the pertinent first ligands in said immobilized standard sequences and immobilized target sequences;
(5) detecting said labeling substance to detect standard sequences and target sequences;
(6) obtaining the ratio of standard sequences in a reference nucleic acid and a subject nucleic acid, calculating a coefficient which corrects detection values of target sequences in the subject nucleic acid, and correcting detection values of target sequences in the subject nucleic acid using the pertinent correction coefficient;
(7) comparing corrected detection values of target sequences in the subject nucleic acid with detection values of target sequences in the reference nucleic acid to confirm an increase/decrease in the quantity of target sequences in a subject nucleic acid relative to target sequences in a reference nucleic acid.

3. A method for analyzing primary structure change of nucleic acid comprising the steps of:
(1) obtaining a reference nucleic acid having a standard sequence and a target sequence whose existence/non-existence or increase/decrease in amount thereof is to be detected, each sequence being bound by a first ligand that differs depending on type of the sequence to which it binds, and being bound by a second ligand that may be identical or different for each type of the sequence;
(2) adding first ligands and second ligands to a subject nucleic acid which has a standard sequence and a target sequence whose existence/non-existence or quantity thereof is unknown, in order to obtain nucleic acids which have a structure in which a standard sequence is bound by a first ligand that differs depending on type of the sequence to which it binds, and a second ligand that may be identical or different for each type of the sequence; and if a target sequence is present in the subject nucleic acid, the target sequence is bound by a first ligand that differs depending on type of the sequence, and a second ligand that may be identical or different for each type of the sequence;
(3) preparing a carrier which supports receptors that specifically bind to said first ligands or second ligands, binding said first ligands or second ligands to the pertinent receptors, and immobilizing said standard sequences and target sequences on the carrier, and causing agglutination of the pertinent immobilized standard sequences and immobilized target sequences by an agglutination reaction of the carrier;
(4) detecting said agglutination product to detect standard sequences and target sequences;
(5) obtaining the ratio of standard sequences in a reference nucleic acid and a subject nucleic acid, calculating a coefficient which corrects detection values of target sequences in the subject nucleic acid, and correcting detection values of target sequences in the subject nucleic acid using the pertinent correction coefficient;
(6) comparing corrected detection values of target sequences in the subject nucleic acid with detection values of target sequences in the reference nucleic acid to confirm an increase/decrease in the quantity of target sequences in a subject nucleic acid relative to target sequences in a reference nucleic acid.

4. A method for analyzing primary structure change of nucleic acid according to any one of the claims 1 to 3, wherein said second ligands are all uniform.

5. A method for analyzing primary structure change of nucleic acid according to any one of the claims 1 to 4, wherein loss of heterozygosity is detected.

6. A method for analyzing primary structure change of nucleic acid according to any one of the claims 1 to 5, wherein there is a plurality of said target sequences.

7. A method for analyzing primary structure change of nucleic acid according to any one of the claims 1 to 6, wherein said standard sequences and/or target sequences have nucleotide sequences whose positions on a chromosome are identified as markers.

8. A method for analyzing primary structure change of nucleic acid according to claim 7, wherein said nucleotide sequences whose positions on a chromosome are identified as markers are one or more types selected from the group consisting of microsatellite markers, SNP markers, STS markers, EST markers and cDNA markers.

9. A method for analyzing primary structure change of nucleic acid according to any one of the claims 1 to 8, wherein one of said first and second ligands contain a primer which can amplify said standard sequences and target sequences by a polymerase reaction, while the remaining one of said first or second ligands contain a probe which can hybridize said standard sequences and target sequences, and wherein said standard sequences and target sequences are amplified by a polymerase reaction using the primer, and the amplified standard sequences and target sequences are detected after hybridizing the probe.

10. A method for analyzing primary structure change of nucleic acid according to claim 9, wherein said polymerase reaction is an isothermal amplification reaction.

11. A method for analyzing primary structure change of nucleic acid according to any one of the claims 1 to 8, wherein said first and second ligands contain a primer capable of amplifying said standard sequences and target sequences by a polymerase chain reaction, and said standard sequences and target sequences are detected after being amplified by a polymerase chain reaction using the primer.

12. A method for analyzing primary structure change of nucleic acid according to any one of the claims 1 to 8, wherein said first and second ligands contain probes having nucleotide sequences which are complementary with a portion of said standard sequences and target sequences, and said probes undergo hybridization and ligation in said standard sequences and target sequences, after which said standard sequences and target sequences which are the ligation products are detected.

13. A method for analyzing primary structure change of nucleic acid according to claim 12, wherein said probes are hybridized after amplifying said standard sequences and target sequences by polymerase reaction.

14. A method for analyzing primary structure change of nucleic acid according to any one of the claims 1 to 13, wherein said first and second ligands are polypeptides consisting of two or more amino acid residues.

15. A method for analyzing primary structure change of nucleic acid according to any one of the claims 1 to 14, which has a process where said carrier is a particulate carrier endowed with magnetism.

16. A method for analyzing primary structure change of nucleic acid according to any one of the claims 1 to 15, wherein the combination of said first ligands and/or second ligands with receptors is a combination of antigen and antibody.

17. A method for analyzing primary structure change of nucleic acid according to claim 16, wherein said antibody is a monoclonal antibody.

18. A method for analyzing primary structure change of nucleic acid according to claim 16, wherein said antibody is an antibody obtained by the phage display method.

19. A method for analyzing primary structure change of nucleic acid according to any one of the claims 1 to 18, wherein the detection values of standard sequences and target sequences of said reference nucleic acid are registered in a database, and correction of detection values of target sequences in the subject nucleic acid and comparison of corrected detection values of target sequences in the subject nucleic acid and detection values of target sequences in the reference nucleic acid are conducted using said database.

20. A method for determining nucleic acid mutations **characterized in that** it is determined that amplification of a target sequence in a chromosome has occurred when a corrected detection value of a target sequence in the subject nucleic acid obtained by the method for analyzing primary structural change of nucleic acid according any one of the claims 1 to 19 is larger than a threshold value.

21. A method for determining nucleic acid mutations **characterized in that** it is determined that loss of a target sequence in a chromosome has occurred when a corrected detection value of a target sequence in the subject nucleic acid obtained by the method for analyzing primary structural change of nucleic acid according any one of the claims 1 to 19 is smaller than a threshold value.


### Sequence Listings

SequenceListing
SEQUENCE LISTING
<110>Olympus Corporation
<120>Analysis Method for The Change of Primary Structure in Nucleic Acid
<130>PC10399
<160>16
<210>1
<211>21
<212>DNA
<213>Artificial Sequence
<220>
<223>Designed DNA fragment to act as a primer in PCR method for amplification of
 standard sequence SEQ ID NO:5.
<400>1
agctcacatt ctagtcagcc t 21

<210>2
<211>16
<212>DNA
<213>Artificial Sequence
<220>
<223>Designed DNA fragment to act as a primer in PCR method for amplification of
 standard sequence SEQ ID NO:5.
<400>2
gccaattccc cagatg 16

<210>3
<211>25
<212>DNA
<213>Artificial Sequence
<220>
<223>Designed DNA fragment to act as a primer in PCR method for amplification of
 target sequence SEQ ID NO:6.
<400>3
aatccctttt ctacctttct atcac 25

<210>4
<211>26
<212>DNA
<213>Artificial Sequence
<220>
<223>Designed DNA fragment to act as a primer in PCR method for amplification of
 target sequence SEQ ID NO:6.
<400>4
gagaggagag ataaaagatg taaatg 26

<210>5
<211>239
<212>DNA
<213>Artificial Sequence
<220>
<223>Designed DNA fragment as standard sequence using for detection of target
sequence SEQ ID NO:6.
<400>5
agctcacatt ctagtcagcc tgagacacac acacacacac acacacacac acacacacac 60
acacacacac aaataaatta tataataatt tagtgtataa acactacaaa ggaacgtcat 120
gtggtagggg tgcaatttta agataatggg aaagactatt ctgtgacatt tgagcacaat 180
ctaaatagag gtctgaagga gttgagtgaa catagcatat gtacatctgg ggaattggc 239

<210>6
<211>166
<212>DNA
<213>Artificial Sequence
<220>
<223>Designed DNA fragment that should be detected as target sequence.
<400>6
aatccctttt ctacctttct atcaccctgc attctcttca gtctctcttt ctccttgcag 60
atagatgata gatagataga tagatagata gatagataga tagataaacg gatagataga 120
taggtagaca gacagatctc catttacatc ttttatctct cctctc 166

SequenceListing

<210>7
<211>80
<212>DNA
<213>Artificial Sequence
<220>
<223>Designed DNA fragment as standard sequence using for detection of target
sequence SEQ ID NO:8.
<400>7
gttgccctgt cctttggata cctcctgact gggaagctgt ctaggccttt atcttactgc 60
agctgttagg cagtcataga 80

<210>8
<211>85
<212>DNA
<213>Artificial Sequence
<220>
<223>Designed DNA fragment that should be detected as target sequence.
<400>8
ggttgacagg caggccacct gtctgacgag atagcrcagg tcaggtgggc tcactctaag 60
cccctgtatt ctcatgacct gtaag 85

<210>9
<211>16
<212>DNA
<213>Artificial Sequence
<220>
<223>Designed DNA fragment for ligation to form standard sequence SEQ ID NO:7.
<400>9
ctgactggga agctgt 16

<210>10
<211>21
<212>DNA
<213>Artificial Sequence
<220>
<223>Designed DNA fragment for ligation to form standard sequence SEQ ID NO:7.
<400>10
ctaggccttt atcttactgc a 21

<210>11
<211>18
<212>DNA
<213>Artificial Sequence
<220>
<223>Designed DNA fragment for ligation to form target sequence SEQ ID NO:8.
<400>11
ctgtctgacg agatagca 18

<210>12
<211>18
<212>DNA
<213>Artificial Sequence
<220>
<223>Designed DNA fragment for ligation to form target sequence SEQ ID NO:8.
<400>12
ctgtctgacg agatagcg 18

<210>13
<211>18
<212>DNA
<213>Artificial Sequence
<220>
<223>Designed DNA fragment for ligation to form target sequence SEQ ID NO:8.
<400>13
caggtcaggt gggctcac 18

<210>14

SequenceListing
<211>23
<212>PRT
<213>Artificial Sequence
<220>
<223>Designed peptide to act as a hapten using for modification of 5'-end of
primer SEQ ID NO:1.
<400>14
Ser Thr Asp Ala Thr Ser Ile Leu Gly Ile Gly Thr Val Leu Asp Gln
1                   5                   10                  15
Ala Glu Thr Ala Gly Ala Arg
            20

<210>15
<211>25
<212>PRT
<213>Artificial Sequence
<220>
<223>Designed peptide to act as a hapten using for modification of 5'-end of
primer SEQ ID NO:3.
<400>15
Pro Pro Gly Ser Val Thr Val Pro His Pro Asn Ile Glu Glu Val Ala
                    5                   10                  15
Leu Ser Thr Thr Gly Glu Ile Pro Phe
            20                  25

<210>16
<211>16
<212>PRT
<213>Artificial Sequence
<220>
<223>Designed peptide to act as a hapten using for modification of 5'-ends of
primer SEQ ID NO:2 and 4.
<400>16
Tyr Tyr Arg Gly Leu Asp Val Ser Val Ile Pro Thr Ser Gly Asp Val
                    5                   10                  15

1